# EUROPÄISCHE PATENTSCHRIFT

(11) **EP 3 393 596 B1**
(45) Veröffentlichungstag und Bekanntmachung des Hinweises auf die Patenterteilung: **08.04.2020**
(21) Anmeldenummer: 16808963.9
(22) Anmeldetag: 01.12.2016
(51) Int. Cl.: A61Q 5/08, A61K 8/73, A61K 8/19, A61K 8/26, A61Q 5/10

(54) **GERUCHSREDUZIERTE BLONDIERUNG**
ODOR-REDUCED BLEACH
COMPOSITION D'ECLAIRCISSEMENT AVEC UN NIVEAU REDUIT DE MAUVAISES ODEURS

(30) Priorität: 21.12.2015 DE 102015226170
(43) Veröffentlichungstag der Anmeldung: 31.10.2018
(73) Patentinhaber: Henkel AG & Co. KGaA, 40589 Düsseldorf (DE)
(72) Erfinder: MANNECK, Hartmut, 23858 Barnitz (DE); HOEPFNER, Stefan, 22523 Hamburg (DE); SCHWEINSBERG, Matthias, 40764 Langenfeld (DE)
(86) Internationale Anmeldenummer: PCT/EP2016/079380
(87) Internationale Veröffentlichungsnummer: WO 2017/108363

(56) Entgegenhaltungen:
- EP-A1- 0 114 414
- WO-A1-94/16672
- DE-A1-102012 223 205

## Beschreibung

### Technischer Hintergrund

Neben der Färbung ist das Aufhellen der eigenen Haarfarbe bzw. das Blondieren der ganz spezielle Wunsch vieler Verbraucher, da eine blonde Haarfarbe als attraktiv und in modischer Hinsicht erstrebenswert betrachtet wird. Sollen Keratinfasern aufgehellt oder gar gebleicht werden, werden die die Keratinfasern färbenden Farbstoffe, beispielsweise das Haar-eigene Melanin, meist oxidativ unter Einsatz von entsprechenden Oxidationsmitteln, wie Wasserstoffperoxid, entfärbt.

Bei der Haarblondierung - insbesondere bei der Haarblondierung in Heimanwendung durch den Verbraucher selbst - tritt das Problem auf, dass natürliche Nuancierungen vollständig überdeckt werden, so dass multitonale Haarblondierungen schwierig zu realisieren sind.

Um dem Haar ein natürlicheres Aussehen zu verleihen, ist es im Stand der Technik bekannt, ungefärbte wie auch bereits gefärbte Haare durch die gezielte Anwendung von Oxidationsmitteln partiell zu entfärben. Die Haarpartien ("Strähnchen"), auf die die Oxidationsmittel aufgetragen werden, bleichen dabei mindestens anteilsweise aus, woraus eine multitonale Haarfarbe resultiert. Auch die Färbung einzelner Haarpartien ("Strähnchen") mit einer anderen Farbe ist im Stand der Technik bekannt. Die Applikation des ggf. farbstoffhaltigen Oxidationsmittels erfolgt dabei mit einer Bürste oder einem Pinsel, wobei die nicht zu behandelnden Haare mit festen flächigen Trennhilfen, insbesondere mit Folien aus festen Materialien, wie Aluminium, Papier oder Styropor und ähnlichen Materialien, oder mit einer so genannten "Strähnchenhaube" vor dem Kontakt mit dem ggf. farbstoffhaltigen Oxidationsmittel geschützt werden. Eine derartige Strähnchenhaube ist beispielsweise in WO 2007/087978 offenbart. Siehe als Stand der Technik auch WO 94/16672 A1, bzw. DE 10 2012 223205 A1.

Bei den Strähnchenfärbeverfahren des Standes der Technik fallen die Folien oder die Strähnchenhaube am Ende des Verfahrens als fester Abfall an, der entsorgt werden muss. Dies ist mit einer entsprechenden Umweltbelastung verbunden. Der Umgang mit den Folien ist teilweise schwierig; z. B. wird der Zugang zu noch nicht behandelten Haaren durch bereits liegende Folien behindert. Darüber hinaus besteht insbesondere bei Folien aus Papier, Plastik und Styropor, die Gefahr, dass sie auf dem behandelten Haar schlecht haften und abrutschen.

Bei einigen Folientypen und Anwendungen kann es zur Überhitzung kommen, weil die Möglichkeit, die Reaktionswärme des exothermen Oxidationsprozesses durch Verdunstung flüchtiger Rezepturbestandteile, wie beispielsweise Wasser, abzuführen, durch die dampfundurchlässigen Folien stark eingeschränkt ist.

Die meisten Folientypen sind nicht durchsichtig. Um - vor allem gegen Ende der vorgeschriebenen Einwirkzeit - die Farbentwicklung kontrollieren zu können, muss die Folie wenigstens teilweise abgewickelt werden, was den Farbentwicklungsprozess stören kann und außerdem im Arbeitsablauf hinderlich ist.

Blondiermittel, insbesondere solche, die eine starke Aufhellung ("high lift") bewirken sollen, enthalten in der Regel größere Mengen an Ammoniumhydroxid als Alkalisierungsmittel. Während der Einwirkzeit, die üblicherweise mindestens 30 Minuten bis 60 Minuten beträgt, entweicht hieraus Ammoniak, was neben der Geruchsbelästigung und Schleimhautreizung auch zu einem Leistungsverlust des Mittels bei der Aufhellleistung führt. Außerdem neigen Blondiermittel, insbesondere, wenn sie nicht von Strähnchenfolien bedeckt sind, sondern großflächig aufgetragen wurden, während dieser relativ langen Einwirkzeit dazu, anzutrocknen, was ebenfalls zu einer verminderten Aufhellleistung führt. Um die Verminderung der Aufhellleistung durch das Antrocknen auszugleichen, wird meist für das Oxidationsmittel eine höhere Wasserstoffperoxidkonzentration gewählt, als eigentlich notwendig wäre. Dies kann wiederum zu stärkeren Strukturschäden an den Keratinfasern und/oder einer stärkeren Reizung der Kopfhaut führen als eigentlich notwendig wäre.

### Stand der Technik

Die Offenlegungsschriften EP2574331A2 und WO2014164213A1 offenbaren Färbeverfahren, die multitonale Färbungen oder Blondierungen in einem Färbeschritt ermöglichen, bei dem einem HaarBlondier- oder -bleichmittel vor der Applikation auf ausgewählte Haarpartien oder Strähnen ein Verdickungsmittel, insbesondere ein anionisches oder kationisches Assoziativpolymer (EP2574331A2), das verdickend wirkt, in höherer Konzentration zugesetzt wird. Das Färbemittel verdickt auf den Haarsträhnen zu einer hoch viskosen Paste, wodurch eine Trennwirkung zu benachbarten Haarpartien oder Strähnen erzielt wird. Der Farbaustausch zwischen benachbarten Strähnen, die mit unterschiedlichen Blondier-oder Bleichmitteln behandelt wurden, wird so minimiert. Über die gesamte Einwirkzeit der Färbemittel von ca. 30 bis 60 Minuten hinweg lässt sich die Farbstoffdiffusion zwischen benachbarten Strähnen, die in physischem Kontakt zueinander stehen, allerdings nicht vollständig vermeiden. Das entsprechende Handelsprodukt für die professionelle Salonanwendung wird daher mit dem Hinweis vermarktet, dass der Farbunterschied zwischen benachbarten Strähnen nicht mehr als 3 Farbtonstufen betragen sollte. Andernfalls würde das Färbeergebnis durch einen sichtbaren Farbaustausch beeinträchtigt.

### Aufgabe

Der vorliegenden Erfindung lag die Aufgabe zugrunde, ein Verfahren bereitzustellen, das multitonale Blondierungen in einem Arbeitsgang ermöglicht. Eine weitere Aufgabe der vorliegenden Erfindung war es, ein möglichst ressourcenschonendes multitonales Blondier- oder Aufhellverfahren bereitzustellen, das ohne festen Folienabfall und möglichst nur mit Materialien aus nachwachsenden Rohstoffen auf nicht-fossiler Rohstoffbasis auskommt. Bei dieser Aufgabenstellung war es besonders wichtig, dass die Folienersatzstoffe ebenso wie die flächigen Folien eine hohe Trennkraft aufweisen, das heißt, eine möglichst saubere Trennung der unterschiedlich gefärbten oder gebleichten Faserpartien oder Fasersträhnen erlauben, ohne dass es zu einem Blondiermittelaustausch zwischen benachbarten Strähnen kommt, wodurch breitere Strähnen als eigentlich beabsichtigt entfärbt würden. Eine weitere Aufgabe der vorliegenden Erfindung war es, ein multitonales Blondier- oder Aufhellverfahren bereitzustellen, das eine vereinfachte optische Kontrolle der Entfärbungsentwicklung ermöglicht.

Der vorliegenden Anmeldung lag weiterhin die Aufgabe zu Grunde, ein Verfahren zur oxidativen Aufhellung oder Blondierung von Keratinfasern bereitzustellen, bei dem das unerwünschte Eintrocknen des Aufhell- oder Färbemittels reduziert wird, wodurch mit geringeren Einsatzkonzentrationen an Wasserstoffperoxid gearbeitet werden kann bei gleichbleibender Aufhellleistung. Der vorliegenden Anmeldung lag weiterhin die Aufgabe zu Grunde, ein Verfahren zur oxidativen Aufhellung oder Blondierung von Keratinfasern bereitzustellen mit reduziertem Kopfhautreizpotenzial. Der vorliegenden Anmeldung lag weiterhin die Aufgabe zu Grunde, ein Verfahren zur oxidativen Aufhellung oder Blondierung von Keratinfasern bereitzustellen, bei dem die unerwünschte Freisetzung von Ammoniak während der Einwirkzeit auf den Keratinfasern reduziert wird.

Prinzipiell ist das erfindungsgemäße Verfahren auch zur monotonalen Farbveränderung, insbesondere zur Aufhellung und/oder Blondierung von Keratinfasern geeignet. Dabei kann das gesamte Keratinfaserensemble, z. B. das gesamte Haupthaar, einheitlich ohne partielle, z.B. strähnenförmige, Variation der Farbtöne und Highlights mit der Blondier- oder Aufhellzusammensetzung beaufschlagt und anschließend der erfindungsgemäßen Filmbildungsprozedur unterzogen werden.

Gegenstand der vorliegenden Anmeldung ist ein Verfahren zur Aufhellung oder Blondierung keratinischer Fasern, das eine verringerte Freisetzung von Ammoniak aufweist und es erlaubt, mit geringeren Einsatzkonzentrationen an Wasserstoffperoxid eine gleichbleibende Aufhellleistung zu erzielen, und das es weiterhin ermöglicht, in einer einzigen Behandlungssitzung das Haar zu blondieren und gleichzeitig eine multitonale Färbung mit verschiedenfarbigen Partien (Strähnchen), insbesondere helleren ("highlights") Partien (Strähnchen), zu erzeugen, wobei auf den Einsatz von festen flächigen Trennhilfen, insbesondere von Folien aus festen Materialien, wie Aluminium, Papier oder Styropor und ähnlichen Materialien verzichtet werden kann.

Hierzu wird dem anwendungsbereiten Blondier- oder Aufhellmittel, das üblicherweise einen stärker alkalischen pH-Wert aufweist, oder seinen einzelnen Komponenten zunächst mindestens ein ausgewähltes Salz eines mehrwertigen Metallions, ausgewählt aus Calcium-, Strontium-, Barium- und Aluminiumsalzen und unter bestimmten Voraussetzungen ausgewählt aus Kaliumsalzen, jeweils mit einer Wasserlöslichkeit bei 20°C von mindestens 500 mg/l, zugemischt, wobei die mehrwertigen Metallionen mit bestimmten Polysacchariden in wässrigem Medium ein Gel oder einen gelierten Film bilden.

Anstatt dieses ausgewählte Salz eines mehrwertigen Metallions dem anwendungsbereiten Blondier-oder Aufhellmittel oder seinen einzelnen Komponenten zuzumischen, kann zunächst auch das anwendungsbereite Blondier- oder Aufhellmittel auf ausgewählte Keratinfaserpartien, insbesondere Haarpartien, oder Keratinfasersträhnen, insbesondere Haarsträhnen, in üblicher Weise aufgetragen und anschließend mit einer wässrigen Lösung des ausgewählten Salzes eines mehrwertigen Metallions besprüht oder bestrichen werden. Anschließend werden die mit dem ausgewählten Salz behandelten Faserpartien oder Strähnen mit einer wässrigen Lösung eines ausgewählten Polysaccharids besprüht oder bestrichen, das mit den ausgewählten Kationen wechselwirkt und zu einem filmförmigen Gel verfestigt wird. Dieser Film wirkt, ähnlich wie die Strähnchenfolien des bekannten Verfahrens, als Trennschicht, die es erlaubt, benachbarte Faserpartien oder Strähnen verschiedenfarbig zu färben oder zu bleichen. Darüber hinaus wirkt dieser Film als durchsichtige Barriere, die die Freisetzung von Ammoniak und das Verdunsten von Wasser verlangsamt und außerdem eine einfache optische Kontrolle der Entwicklung des Blondier- oder Aufhelleffekts während der Einwirkzeit ermöglicht.

Unter den Begriffen "keratinische Fasern" und "Keratinfasern" sind erfindungsgemäß bevorzugt menschliche Haare, daneben aber auch Pelze, Wolle und Federn zu verstehen.

Unter "anschließend" wird erfindungsgemäß bevorzugt ein Zeitraum von 1 bis 600 Sekunden verstanden.

Gegenstand der Erfindung sind ein Verfahren zur Aufhellung und Blondierung keratinischer Fasern sowie ein Kit dem strikten Wortlaut der Ansprüche gemäss.

Gegenstand der vorliegenden Erfindung ist ein Verfahren zur Aufhellung und/oder Blondierung keratinischer Fasern, gekennzeichnet durch folgende Verfahrensschritte
I. Bereitstellen einer Blondier- oder Aufhellzusammensetzung (A), enthaltend
   a)i. mindestens ein Alkalisierungsmittel,
   a)ii. optional mindestens einen Konsistenzgeber und
   a)iii. optional mindestens ein Lösemittel, ausgewählt aus Wasser und mindestens einem organischen Lösemittel, das bevorzugt ausgewählt ist aus einem oder mehreren ein- oder mehrwertigen Alkoholen mit 2 bis 9 Kohlenstoffatomen, Polyethylenglycolen mit 2 bis 20 Ethylenglycoleinheiten sowie Mischungen dieser Lösemittel, und
   a)iv. optional mindestens einen direktziehenden Haarfarbstoff,
   a)v. wobei die Blondier- oder Aufhellzusammensetzung (A), sofern sie, bezogen auf ihr Gewicht, mehr als 7 Gew.-% Wasser enthält, einen pH-Wert im Bereich von 6,0 bis 11,5 aufweist, gemessen bei 20°C, und sofern die Zusammensetzung (A), bezogen auf ihr Gewicht, 0 bis 7 Gew.-% Wasser enthält, ihre 30 Gew.-%ige Dispersion in Wasser einen pH-Wert im Bereich von 6,0 bis 12,0, bevorzugt 7,5 - 11,5, besonders bevorzugt 8 bis 11,0, aufweist, jeweils gemessen bei 20°C;
II. Bereitstellen einer Oxidationszusammensetzung (B), enthaltend
   b)i. 1 - 18 Gew.-% Wasserstoffperoxid, bezogen auf das Gewicht der Zusammensetzung (B), und
   b)ii. Wasser,
   b)iii. wobei die Oxidationszusammensetzung (B) einen pH-Wert im Bereich von einen pH-Wert im Bereich von 2,5 - 6,5, bevorzugt im Bereich von 3 - 5,5, besonders bevorzugt im Bereich von 3,5 bis 5,0, aufweist, jeweils bei 20°C gemessen;
c. Bereitstellen einer Polysaccharid-Zusammensetzung (D), enthaltend
   c)i. mindestens ein Polysaccharid, das mit Calciumionen in wässrigem Medium ein Gel bilden kann und ausgewählt ist aus Alginsäure, kappa-Carragheenan, iota-Carragheenan und Pektin, und/oder mindestens einem Salz der vorgenannten Polysaccharide, ausgewählt aus den Alkalimetall-, den Ammonium-, den Mono-, Di- und Trialkylammonium-, den Mono-, Di- und Trialkanolammonium- und den Magnesiumsalzen, bevorzugt in einer Gesamtmenge von 0,1 - 5 Gew.-%, bevorzugt 0,5 - 2 Gew.-%, besonders bevorzugt 0,5 - 2,5 Gew.-%, jeweils bezogen auf das Gewicht der Zusammensetzung (D), wobei im Falle von Salzen des kappa-Carragheenans dessen Alkalimetall-Salz nur aus Lithium- und Natriumsalzen ausgewählt ist,
   c)ii. 50 - 99,9, bevorzugt 60 bis 95, besonders bevorzugt 80 bis 90 Gew.-% Wasser, jeweils bezogen auf das Gewicht der Zusammensetzung (D),
   c)iii. optional mindestens ein Alkalisierungsmittel, das bevorzugt ausgewählt ist aus pulverförmigen Natriumsilikaten, insbesondere pulverförmigen Natriummetasilikaten,
   c)iv. optional mindestens ein Polymer, enthaltend (Meth)Acrylsäure-, (Meth)Acrylsäureester-, (Meth)Acrylamid- und/oder 2-Acrylamido-2-methylpropansulfonsäure-Monomere, bevorzugt in einer Gesamtmenge von 0,05 - 3 Gew.-%, bevorzugt 0,2 - 0,5 Gew.-%, bezogen auf das Gewicht der Zusammensetzung (D),
   c)v. optional mindestens ein Öl in einer Gesamtmenge von 0,1 bis 10 Gew.-%, bevorzugt 0,3 bis 5 Gew.-%, besonders bevorzugt 1 bis 3 Gew.-%, jeweils bezogen auf das Gewicht der Polysaccharid-Zusammensetzung (D),
   wobei optional die Polysaccharid-Zusammensetzung (D) durch Vermischen einer festen, bevorzugt pulverförmigen, Polysaccharid-Zusammensetzung (D'), enthaltend c)i und optional c)iii und/oder optional c)iv, mit der Komponente c)ii und optional mit der Komponente c)v *in situ* oder 0,01 bis 24 Stunden vor Durchführung des erfindungsgemäßen Farbveränderungsverfahrens hergestellt wird,
   anschließend, bevorzugt 1 bis 600 Sekunden danach,
d. Herstellen einer Mischung (M) aus (A) und (B), die einen pH-Wert im Bereich von 6,0 bis 11 aufweist, jeweils gemessen bei 20°C; nach 1 bis 600 Sekunden dann
e. Applikation zumindest einer Teilmenge von (M) auf mindestens eine zu blondierende und/oder aufzuhellende Keratinfaserpartie oder Applikation des Mittels (M) auf das gesamte zu behandelnde Keratinfaserensemble;
   1 bis 600 Sekunden, bevorzugt 5 bis 300 Sekunden, besonders bevorzugt 5 bis 60 Sekunden danach
f. Applizieren, bevorzugt Aufsprühen, der Polysaccharid-Zusammensetzung (D), auf die mit (M) beaufschlagte(n) Keratinfaserpartie(n) oder das mit (M) beaufschlagte Keratinfaserensemble;
g. ggf. Wiederholen der Schritte e. und f. im gewünschten Umfang,
h. für eine Zeit von 0,1 bis 60 Minuten, bevorzugt 1 bis 50 Minuten, besonders bevorzugt 10 bis 45 Minuten, außerordentlich bevorzugt 30 bis 45 Minuten Belassen auf den keratinischen Fasern, anschließend Spülen der keratinischen Fasern mit Wasser und ggf. einem Reinigungsmittel, ggf. Nachbehandlung der Fasern mit einem Konditioniermittel und anschließendes Trocknen,
das dadurch gekennzeichnet ist, dass das Verfahren die Applikation von mindestens einem Salz eines mehrwertigen Metallions, ausgewählt aus Calcium-, Strontium-, Barium- und Aluminiumsalzen mit einer Wasserlöslichkeit bei 20°C von mindestens 500 mg/l, umfasst, sowie für den Fall, dass das mit Calciumionen Gel bildende Polysaccharid kappa-Carragheenan oder eines seiner Salze umfasst, das Verfahren die Applikation von mindestens einem Salz, ausgewählt aus Kalium-, Calcium-, Strontium-, Barium- und Aluminiumsalzen mit einer Wasserlöslichkeit bei 20°C von mindestens 500 mg/l enthält umfasst,
wobei diese Salze nicht in der Polysaccharid-Zusammensetzung (D) enthalten sind.

Die vorliegende Erfindung bezieht sich auf die oxidative Farbveränderung, insbesondere die Aufhellung und/oder Blondierung, keratinischer Fasern, insbesondere von Haaren. Da bei der Behandlung von keratinischen Fasern, insbesondere Haaren, mit Oxidationsmitteln, insbesondere mit Wasserstoffperoxid, der fasereigene Farbstoff Melanin zu einem gewissen Grad zerstört wird, werden die Fasern/Haare zwangsläufig aufgehellt, ändern also ihre Farbe auch ohne die Gegenwart eines Farbstoffs. Daher umfasst der Begriff "Farbveränderung" im Sinne der vorliegenden Anmeldung sowohl die Aufhellung als auch die Färbung mit einem oder mehreren Farbstoffen.

Für die Farbveränderung von menschlichen Haaren kennt der Fachmann verschiedene Verfahren. Im allgemeinen werden für die Färbung menschlicher Haare entweder direktziehende Farbstoffe oder Oxidationsfarbstoffe verwendet, die durch oxidative Kupplung einer oder mehrerer Entwicklerkomponenten untereinander oder mit einer oder mehreren Kupplerkomponenten entstehen. Kuppler- und Entwicklerkomponenten werden auch als Oxidationsfarbstoffvorprodukte bezeichnet. Die mit Oxidationsfarben erzielten Färbungen werden zumeist als permanente oder semipermanente Färbungen bezeichnet. Als Oxidationsmittel enthalten diese Mittel zumeist Wasserstoffperoxid. Da Wasserstoffperoxid im alkalischen pH-Bereich nur unzureichend lagerstabil ist, umfassen oxidative Färbemittel üblicherweise zwei Komponenten, die unmittelbar, das heißt, in einem Zeitraum von 1 bis 600 Sekunden vor der Anwendung, miteinander vermischt werden. Die eine Komponente, im Folgenden als "Oxidationszusammensetzung (B)" bezeichnet, enthält Wasserstoffperoxid in wässriger Lösung oder Emulsion, wobei diese Zusammensetzung zur Stabilisierung des Wasserstoffperoxids einen sauren pH-Wert im Bereich von 2,5 bis 6,5, bevorzugt im Bereich von 3 - 5,5, besonders bevorzugt im Bereich von 3,5 bis 5,0, aufweist, jeweils bei 20°C gemessen.

Die zweite Komponente, im Folgenden als "Blondier- oder Aufhellzusammensetzung (A)" bezeichnet, enthält ein oder mehrere Alkalisierungsmittel in einer solchen Menge, dass die Anwendungsmischung aus beiden Komponenten einen pH-Wert im Bereich von 8 - 11 aufweist, jeweils bei 20°C gemessen. Die erfindungsgemäß verwendeten Blondier- oder Aufhellzusammensetzungen (A) enthalten kein Oxidationsfarbstoffvorprodukt und nur relativ geringe Mengen an direktziehenden Farbstoffen. Letztere dienen zur Kaschierung unerwünschter Farbtöne, die bei der Melaninoxidation entstehen können. Daneben gibt es auch Blondierkits und Blondierverfahren, bei denen die Anwendungsmischung aus beiden Komponenten einen pH-Wert im Bereich von etwa 6 bis 7,9 aufweist; die Aufhellergebnisse dieser so genannten "sauren" Blondierungen erreichen allerdings häufig nicht die Güte, die mit stärker alkalischen Anwendungsmischungen erzielt werden. Für das Blondier- oder Aufhellergebnis ist es wichtig, dass das anwendungsbereite Blondier- oder Aufhellmittel, das durch Vermischen der erfindungsgemäß verwendeten Blondier- oder Aufhellzusammensetzung (A) mit der Zusammensetzung (B) erhalten wird, einen pH-Wert im Bereich von 6,5 bis 11,0, bevorzugt 8 bis 10,5, besonders bevorzugt 8,5 bis 9,5, aufweist, jeweils gemessen bei 20°C. Bei diesen pH-Werten öffnet sich die äußere Keratinfaserschicht optimal zur Aufnahme des Oxidationsmittels und entfaltet sich die oxidative Wirkung des Wasserstoffperoxids und ggf. weiterer Peroxidverbindungen optimal.

### Gel bildendes Salz

Ein wesentlicher Bestandteil erfindungsgemäßer und erfindungsgemäß bevorzugter Färbeverfahren ist mindestens ein Salz eines mehrwertigen Metallions, ausgewählt aus Calcium-, Strontium-, Barium- und Aluminiumsalzen mit einer Wasserlöslichkeit bei 20°C von mindestens 500 mg/l und unter bestimmten Bedingungen auch ausgewählt aus Kaliumsalzen mit einer Wasserlöslichkeit bei 20°C von mindestens 500 mg/l, das nach dem Auftragen der Mischung (M) aus Blondier- oder Aufhellzusammensetzung (A) und Oxidationszusammensetzung (B) auf die Keratinfasern in Kontakt mit mindestens einem Polysaccharid gebracht wird, das mit Calciumionen ein Gel bilden kann. Dadurch bildet sich ein filmförmiges Gel auf der Mischung (M), das diese vor dem Antrocknen schützt, die Freisetzung von Ammoniak reduziert und gewünschtenfalls als Strähnchenfolie wirkt. Bevorzugt ist dieses mit Calciumionen gelierende Polysaccharid ausgewählt aus Alginsäure, kappa-Carragheenan, iota-Carragheenan und Pektin und/oder mindestens einem Salz der vorgenannten Polysaccharide, das wiederum ausgewählt ist aus den Alkalimetall-, den Ammonium-, den Mono-, Di- und Trialkylammonium-, den Mono-, Di- und Trialkanolammonium- und den Magnesiumsalzen, wobei im Falle von Salzen des kappa-Carragheenans dessen Alkalimetall-Salz nur aus Lithium- und Natriumsalzen ausgewählt ist.

Das mindestens eine Salz d)i weist bei 20°C eine Wasserlöslichkeit von mindestens 500 mg/l, bevorzugt von mindestens 40 g/l, besonders bevorzugt von mindestens 200 g/l, außerordentlich bevorzugt von mindestens 350 g/l, auf. Das erfindungsgemäß außerordentlich bevorzugte Calciumchlorid hat eine Wasserlöslichkeit von 740 g/l bei 20°C. Andere erfindungsgemäß besonders bevorzugte Salze d)i sind ausgewählt aus Calciumacetat (Wasserlöslichkeit 374 g/l), Calciumlactat, Calciumgluconat, Calciumgluconatlactat. Ebenfalls bevorzugt sind Aluminiumchlorid, Aluminiumhydroxychlorid, Aluminiumsulfat, Aluminiumlactat, Aluminiumacetat, Aluminiumgluconat. Für die Gelierung von kappa-Carragheenan sind Kaliumchlorid, Kaliumacetat, Kaliumlactat, Kaliumgluconat und Kaliumsulfat bevorzugt, wobei Kaliumchlorid, Kaliumlactat, und Kaliumgluconat besonders bevorzugt sind. Geeignet sind weiterhin Strontiumchlorid und Bariumchlorid. Bevorzugt können auch Mischungen der vorgenannten Salze enthalten sein.

### Polysaccharid, ausgewählt aus Alginsäure, kappa-Carragheenan, iota-Carraqheenan und Pektin, und/oder mindestens ein Salz dieser Polysaccharide

Ein weiterer wesentlicher Bestandteil erfindungsgemäßer und erfindungsgemäß bevorzugter Aufhell- und Blondierverfahren ist mindestens ein Polysaccharid, das mit Calciumionen in wässrigem Medium ein Gel bildet. Erfindungsgemäß bevorzugte Polysaccharide, die mit Calciumionen in wässrigem Medium ein Gel bilden können, sind ausgewählt aus Alginsäure, kappa-Carragheenan, iota-Carragheenan und Pektin sowie mindestens einem Salz der vorgenannten Polysaccharide, das ausgewählt aus den Alkalimetall-, den Ammonium-, den Mono-, Di- und Trialkylammonium-, den Mono-, Di- und Trialkanolammonium- und den Magnesiumsalzen, wobei im Falle von Salzen des kappa-Carragheenans dessen Alkalimetall-Salz nur aus Lithium- und Natriumsalzen ausgewählt ist. Wässrige Lösungen dieser Polysaccharide und Polysaccharidsalze zeigen je nach Konzentration eine nur leicht erhöhte Viskosität, bilden aber noch keine festen Gele.

Besonders bevorzugt sind Alginsäure, Natriumalginat, Ammoniumalginat, Magnesiumalginat, Monoethanolammoniumalginat, kappa-Carragheenan, das Lithiumsalz von kappa-Carragheenan, das Natriumsalz von kappa-Carragheenan, iota-Carragheenan, das Lithiumsalz von iota-Carragheenan, das Natriumsalz von iota-Carragheenan, das Kaliumsalz von iota-Carragheenan, das Ammoniumsalz von iota-Carragheenan, Pektin, Natriumpektinat, Ammoniumpektinat, Kaliumpektinat und Magnesiumpektinat, sowie Mischungen dieser Substanzen. Erfindungsgemäß außerordentlich bevorzugt sind Mischungen aus Alginsäure und Natriumalginat, insbesondere Mischungen aus Alginsäure und Natriumalginat im Gewichtsverhältnis von Alginsäure zu Natriumalginat im Bereich von 1:2 bis 2:1, bevorzugt im Bereich von 0,8 bis 1,25, besonders bevorzugt im Gewichtsverhältnis 1:1.

Die vorstehend genannten Polysaccharide und/oder die genannten Salze zeigen ein weitgehend pHunabhängiges Verdickungsverhalten. Allerdings gelieren sie im Kontakt mit bestimmten mehrwertigen Ionen, wie Calcium-, Strontium-, Barium- und Aluminiumionen. Darüber hinaus geliert kappa-Carragheenan auch im Kontakt mit Kaliumionen.

Dieses Verhalten wird im Sinne der vorliegenden Anmeldung dazu genutzt, auf ausgewählten Keratinfaserpartien oder Keratinfasersträhnen nach der Applikation des Blondier- oder Aufhellmittels gezielt einen Folien-ähnlichen Film zu erzeugen, der zum einen das Antrocknen der Zusammensetzung während der Einwirkzeit und die Freisetzung von Ammoniak reduziert und zum anderen den Austausch mit dem auf benachbarten Strähnen applizierten Blondier- oder Aufhellmittel auch bei direktem Kontakt verhindert oder zumindest stark reduziert. Um diesen Film zu erzeugen, gibt es drei Ausführungsformen des erfindungsgemäßen Verfahrens.

In der ersten bevorzugten Ausführungsform des erfindungsgemäßen Verfahrens ist mindestens eines der vorstehend genannten Salze eines mehrwertigen Metallions, ausgewählt aus Calcium-, Strontium-, Barium- und Aluminiumsalzen mit einer Wasserlöslichkeit bei 20°C von mindestens 500 mg/l und unter bestimmten Bedingungen auch ausgewählt aus Kaliumsalzen mit einer Wasserlöslichkeit bei 20°C von mindestens 500 mg/l, in einer der Zusammensetzungen (A) oder (B) oder in beiden Zusammensetzungen (A) und (B) enthalten und/oder wird der anwendungsbereiten Blondier-oder Aufhellmittelmischung aus (A) und (B) über eine dritte Komponente) zugemischt. Wenn die Keratinfasersträhnen ausreichend mit dem Salz-haltigen Blondier- oder Aufhellmittel beaufschlagt sind, wird eine Polysaccharid-Zusammensetzung (D) appliziert, die mindestens ein Polysaccharid enthält, das mit Calciumionen in wässrigem Medium ein Gel bilden kann. Erfindungsgemäß bevorzugte Polysaccharide, die mit Calciumionen in wässrigem Medium ein Gel bilden können, sind ausgewählt aus Alginsäure, kappa-Carragheenan, iota-Carragheenan und Pektin sowie mindestens einem Salz der vorgenannten Polysaccharide, das ausgewählt aus den Alkalimetall-, den Ammonium-, den Mono-, Di- und Trialkylammonium-, den Mono-, Di- und Trialkanolammonium- und den Magnesiumsalzen, wobei im Falle von Salzen des kappa-Carragheenans dessen Alkalimetall-Salz nur aus Lithium- und Natriumsalzen ausgewählt ist. Die Polysaccharid-Zusammensetzung (D) kann zum Beispiel mit einem Pinsel oder einem vergleichbaren Applikator auf die Keratinfasern aufgetragen oder aber aufgesprüht werden. Durch die Fällungsreaktion zwischen dem Polysaccharid oder seinem löslichen Salz und den Gel bildenden Kationen aus einer der Zusammensetzungen (A) oder (B) bildet sich in situ auf den mit dem Blondier- oder Aufhellmittel beaufschlagten Keratinfasern ein filmförmiges Gel aus, das als Trennschicht analog zu einer Strähnchenfolie fungiert.

In einer zweiten, besonders bevorzugten, Ausführungsform des erfindungsgemäßen Verfahrens liegt mindestens eines der vorstehend genannten Salze eines mehrwertigen Metallions, ausgewählt aus Calcium-, Strontium-, Barium- und Aluminiumsalzen mit einer Wasserlöslichkeit bei 20°C von mindestens 500 mg/l und unter bestimmten Bedingungen auch ausgewählt aus Kaliumsalzen mit einer Wasserlöslichkeit bei 20°C von mindestens 500 mg/l, in Pulverform vor und wird der Blondier- oder Aufhellzusammensetzung (A), die, wie nachstehend erläutert, bevorzugt eine pulverförmige Blondier- oder Aufhellzusammensetzung (A-2) darstellt, zugesetzt, bevor diese mit der Oxidationsmischung (B) zum Blondier- oder Aufhellmittel (M) vermischt wird. Die Mischung wird anschließend auf die Keratinfasersträhnen aufgetragen. Anschließend wird die Polysaccharid-Zusammensetzung (D) aufgetragen. Dies kann mit einem Pinsel oder einem vergleichbaren Applikator, erfolgt bevorzugt aber durch Aufsprühen auf die Keratinfasern.

In einer dritten spezifischen Ausführungsform des erfindungsgemäßen Verfahrens liegt mindestens eines der vorstehend genannten Polysaccharide und/oder mindestens eines der vorstehend genannten Salze eines mehrwertigen Metallions, ausgewählt aus Calcium-, Strontium-, Barium- und Aluminiumsalzen mit einer Wasserlöslichkeit bei 20°C von mindestens 500 mg/l und unter bestimmten Bedingungen auch ausgewählt aus Kaliumsalzen mit einer Wasserlöslichkeit bei 20°C von mindestens 500 mg/l, in Form einer wässrigen Lösung als Gelierzusammensetzung (C) vor und wird, ähnlich wie die vorstehend erläuterte Polysaccharid-Zusammensetzung (D), auf die ausreichend mit Blondier- oder Aufhellmittel beaufschlagten Keratinfasersträhnen aufgetragen. Dies kann ebenfalls mit einem Pinsel oder einem vergleichbaren Applikator, aber auch durch Aufsprühen auf die Keratinfasern erfolgen. Bevorzugt wird erst die wässrige Zubereitung des Polysaccharids, das mit Calciumionen gelieren kann, appliziert. Anschließend wird Gelierzusammensetzung (C) aufgetragen.

Für die drei vorgenannten bevorzugten Ausführungsformen des erfindungsgemäßen Aufhell- oder Blondierverfahrens ist es besonders bevorzugt, dass das Gewichtsverhältnis von Polysaccharid-Zusammensetzung (D) zur Mischung (M) aus Blondier- oder Aufhellzusammensetzung (A) und Oxidationszusammensetzung (B) im Bereich von 1 : 20 bis 1:2, bevorzugt im Bereich von 1:10 bis 1:5 liegt.

Für das dritte der drei vorgenannten bevorzugten erfindungsgemäßen Aufhell- oder Blondierverfahren ist es weiterhin besonders bevorzugt, dass das Gewichtsverhältnis von Polysaccharid-Zusammensetzung (D) zur Gelierzusammensetzung (C) im Bereich von 0,2 : 1 bis 2:1, bevorzugt im Bereich von 0,5 : 1 bis 1,5 : 1 liegt und besonders bevorzugt 1:1 beträgt.

Für das dritte der drei vorgenannten bevorzugten erfindungsgemäßen Aufhell- oder Blondierverfahren ist es weiterhin besonders bevorzugt, dass das Gewichtsverhältnis von Gelierzusammensetzung (C) zur Mischung (M) aus Blondier- oder Aufhellzusammensetzung (A) und Oxidationszusammensetzung (B) im Bereich von 1 : 20 bis 1:2, bevorzugt im Bereich von 1:10 bis 1:5 liegt.

Wenn nach Ablauf der empfohlenen Einwirkzeit die Blondier- oder Aufhellreaktion beendet ist, kann das gesamte Haarbehandlungsmittel in einem einzigen Arbeitsgang mit Wasser und optional einem Shampoo ausgewaschen werden. Das zeitraubende Abwickeln, wie es bei bekannten Strähnchenfolien nötig ist, entfällt vorliegend. Da die erfindungsgemäß verwendeten Polysaccharide natürlich vorkommende Biopolymere darstellen, sind sie ohne Probleme in Kläranlagen biologisch abbaubar. Sie sind also deutlich einfacher zu entsorgen als flächenförmige Folienmaterialien.

### Blondier- oder Aufhellzusammensetzung (A)

Die Blondier- oder Aufhellzusammensetzung (A) kann fest, insbesondere pulverförmig, sein, aber auch flüssig (0 bis 500 mPas bei 20°C), mittelviskos (> 400 bis 4000 mPas bei 20°C), cremeförmig (> 4000 bis 40.000 mPas bei 20 °C) oder pastenförmig (> 40.000 bis 4.000.000 mPas bei 20°C). Feste Blondier- oder Aufhellzusammensetzungen (A) können auch in Tablettenform oder granuliert vorliegen.

### Aufhellzusammensetzungen (A-1) und (A-2)

In einer ersten bevorzugten Ausführungsform der Erfindung ist die Zusammensetzung (A) ein Mittel, das nach Vermischen mit einer Wasserstoffperoxid-haltigen wässrigen Zusammensetzung (B) zur Aufhellung und/oder Blondierung von Keratinfasern, insbesondere von Humanhaar, dient.

In einer ersten besonders bevorzugten Ausführungsform der Erfindung ist die Aufhellzusammensetzung (A-1) flüssig (0 bis 500 mPas bei 20°C), mittelviskos (> 400 bis 4000 mPas bei 20°C) oder cremeförmig (> 4000 bis 40.000 mPas bei 20 °C) und enthält Wasser in einer Menge von mehr als 7 Gew.-% bis 90 Gew.-%, bevorzugt 20 bis 85 Gew.-%, besonders bevorzugt 40 bis 75 Gew.-%, jeweils bezogen auf das Gewicht der Aufhellzusammensetzung (A-1).

In einer zweiten besonders bevorzugten Ausführungsform der Erfindung ist die Aufhellzusammensetzung (A-2) fest, insbesondere pulverförmig, kann aber auch in Tablettenform oder granuliert vorliegen, und enthält 0 bis weniger als 7 Gew.-% Wasser, bezogen auf das Gewicht der jeweiligen Aufhellzusammensetzung (A-2).

### pH-Wert der Blondier- oder Aufhellzusammensetzungen (A-1)

Die Blondier- oder Aufhellzusammensetzungen (A-1) weisen einen pH-Wert im Bereich von 6,5 bis 12,0, bevorzugt 8 - 11,5, besonders bevorzugt 8,5 bis 11,0, auf, jeweils gemessen bei 20°C.

Die Blondier- oder Aufhellzusammensetzungen (A-2) liegen fest, bevorzugt pulverförmig, oder pastenförmig vor und enthalten kein oder nur bis ca. 7 Gew.-% Wasser. Daneben enthalten sie so viel Alkalisierungsmittel, dass ihre 30 Gew.-%ige Dispersion in Wasser einen pH-Wert im Bereich von 6,0 bis 12,0, bevorzugt 7,5 - 11,5, besonders bevorzugt 8 bis 11,0, aufweist, jeweils gemessen bei 20°C.

### Alkalisierungsmittel

Die Blondier- oder Aufhellzusammensetzungen (A), nämlich (A-1) und (A-2) enthalten mindestens ein Alkalisierungsmittel, das ausgewählt ist aus Ammoniumhydroxid, Monoethanolamin und Natriumsilikaten sowie Mischungen hiervon.

Bevorzugt enthalten die Aufhellzusammensetzung (A-1) Ammoniumhydroxid, also Ammoniak in Form seiner wässrigen Lösung. Bei entsprechenden wässrigen Ammoniak-Lösungen kann es sich um 10 bis 35 prozentige Lösungen handeln (berechnet in Gew.-%, 100 g wässrige Ammoniaklösung enthalten dementsprechend 10 bis 35 g Ammoniak). Bevorzugt wird Ammoniak in Form einer 20 bis 30 Gew.-%igen Lösung, besonders bevorzugt in Form einer 25 Gew.-%igen Lösung eingesetzt.

In einer besonders bevorzugten Ausführungsform sind die erfindungsgemäß verwendeten Zusammensetzungen (A-1) dadurch gekennzeichnet, dass sie Ammoniumhydroxid in einer Menge von 0,20 bis 18,0 Gew.-%, bevorzugt von 1,0 bis 15 Gew.-%, weiter bevorzugt von 2,0 bis 12,0 Gew.-% und besonders bevorzugt von 3,0 bis 9 Gew.-% - bezogen auf das Gewicht der erfindungsgemäß verwendeten Zusammensetzung (A) - enthalten.

Zusätzlich zu bzw. anstelle von Ammoniumhydroxid enthalten bevorzugte erfindungsgemäß verwendete Zusammensetzungen (A), insbesondere die erfindungsgemäß verwendeten Zusammensetzungen (A-1), Monoethanolamin.

Zur Erzielung einer maximalen Geruchsminimierung und zur Optimierung der Echtheitseigenschaften ist Monoethanolamin in einer Gesamtmenge von 0,2 - 9,0 Gew.-%, bevorzugt von 1,0 - 7 Gew.-%, weiter bevorzugt von 2,0 bis 6,0 Gew.-% und besonders bevorzugt von 3,0 bis 5,5 Gew.-% - bezogen auf das Gewicht der erfindungsgemäß verwendeten Zusammensetzung (A) - enthalten. Zur Erzielung einer maximalen Geruchsminimierung und zur Optimierung der Echtheitseigenschaften enthalten die erfindungsgemäß verwendeten Zusammensetzungen (A-1) Monoethanolamin in einer Gesamtmenge von 0,2 - 9,0 Gew.-%, bevorzugt von 1,0 - 7 Gew.-%, weiter bevorzugt von 2,0 bis 6,0 Gew.-% und besonders bevorzugt von 3,0 bis 5,5 Gew.-%, jeweils bezogen auf das Gewicht der erfindungsgemäß verwendeten Zusammensetzungen (A-1).

Bei Natriumsilikaten im Sinne der vorliegenden Erfindung handelt es sich um chemische Verbindungen, die sich aus Natriumoxid und Siliciumdioxid zusammensetzen und die in verschiedenen molaren Verhältnissen (Monosilikat, Metasilikat und Polysilikat) vorkommen können. Ein Beispiel für ein Natriumsilikat ist das Natriumsalz der Orthokieselsäure mit der Summenformel Na₄SiO₄, das auch als Natriumorthosilikat bezeichnet wird.

Weitere Beispiele für geeignete Natriumsilikate sind das Dinatriummetasilikat bzw. Natriummetasilikat mit der Summenformel Na₂SiO₃, das Dinatriumdisilikat mit der Summenformel Na₂Si₂O₅ oder das Dinatriumtrisilikat mit der Summenformel Na₂Si₃O₇.

Silikate in amorpher Form können durch das Zusammenschmelzen von Siliciumdioxid und Alkalioxid in molaren Verhältnissen zwischen 1:1 und 4:1 hergestellt werden. Die so erhaltenen Feststoffe werden bei etwa 150 °C und 5 bar Dampfdruck gelöst, um eine Lösung der Natriumsilikate in Wasser zu erhalten, bei diesen entsprechenden Lösungen handelt es sich um Alkaliwassergläser.

Als Alkaliwassergläser werden aus einer Schmelze erstarrte, glasartige (amorphe) Natriumsilikate oder ihre wässrigen Lösungen bezeichnet. Diese nennt man auch Natronwasserglas. Auch Natronwassergläser sind innerhalb dieser Erfindung von der Definition der Natriumsilikate umfasst.

Die molare Zusammensetzung bei Wassergläsern beträgt üblicherweise 2 bis 4 mol SiO₂ auf 1 mol Alkalioxid (Na₂O).

Ein Beispiel für ein bevorzugtes Natriumsilikat ist Natronwasserglas, das in Form seiner wässrigen Lösung vorliegt, einen Na₂O-Gehalt von 7,5 bis 8,8 Gew.-% und einen SiO2 Gehalt von 25,0 bis 28,5 Gew.-% besitzt und das die CAS-Nr. 1344-09-5 (Chemical Abstracts Number) hat.

Weitere erfindungsgemäß bevorzugt verwendete Zusammensetzungen (A) enthalten als Alkalisierungsmittel mindestens ein Natriumsilikat, bevorzugt Natriummetasilikat oder Natronwasserglas, in einer Gesamtmenge von 0,1 bis 25 Gew.-%, bevorzugt 0,5 bis 20 Gew.-%, besonders bevorzugt 1 bis 17 Gew.-%, außerordentlich bevorzugt 2 bis 9 Gew.-%, jeweils bezogen auf das Gewicht der erfindungsgemäß verwendeten Zusammensetzung (A).

Der Einsatz von Natriumsilikat als Alkalisierungsmittel ist insbesondere für die erfindungsgemäß verwendeten Zusammensetzungen (A-2) bevorzugt.

Weitere erfindungsgemäß bevorzugt verwendete Zusammensetzungen (A-2) enthalten als Alkalisierungsmittel mindestens ein Natriumsilikat, bevorzugt Natriummetasilikat oder Natronwasserglas, in einer Gesamtmenge von 0,1 bis 25 Gew.-%, bevorzugt 0,5 bis 20 Gew.-%, besonders bevorzugt 1 bis 17 Gew.-%, außerordentlich bevorzugt 2 bis 9 Gew.-%, jeweils bezogen auf das Gewicht der erfindungsgemäß verwendeten Zusammensetzung (A-2).

Weitere erfindungsgemäß bevorzugt verwendete Zusammensetzungen (A), insbesondere die Zusammensetzungen (A-2), enthalten als Alkalisierungsmittel mindestens ein Alkalimetall- oder Erdalkalimetallcarbonat, das bevorzugt ausgewählt ist aus Natriumcarbonat, Kaliumcarbonat und Magnesiumcarbonat, sowie Mischungen dieser Carbonate. Das mindestens eine Alkalimetall- oder Erdalkalimetallcarbonat ist bevorzugt in einer Gesamtmenge von 0,1 bis 25 Gew.-%, bevorzugt 0,5 bis 20 Gew.-%, besonders bevorzugt 1 bis 10 Gew.-%, enthalten, jeweils bezogen auf das Gewicht der erfindungsgemäß verwendeten Zusammensetzung (A). Das mindestens eine Alkalimetall- oder Erdalkalimetallcarbonat ist bevorzugt in einer Gesamtmenge von 0,1 bis 25 Gew.-%, bevorzugt 0,5 bis 20 Gew.-%, besonders bevorzugt 1 bis 10 Gew.-%, enthalten, jeweils bezogen auf das Gewicht der erfindungsgemäß verwendeten Zusammensetzung (A-2).

Weiterhin können andere Alkalisierungsmittel, wie Kaliumhydroxid (KOH) und Natriumhydroxid (NaOH) enthalten sein, meist in einer Gesamtmenge von 0,05 bis 1,5 Gew.-%, bevorzugt 0,1 bis 0,6 Gew.-%, jeweils bezogen auf das Gewicht der erfindungsgemäß verwendeten Zusammensetzung (A).

Auch basische Aminosäuren, insbesondere Arginin, Lysin oder Histidin sowie Mischungen dieser Aminosäuren, insbesondere Mischungen aus Arginin und Lysin, können in bevorzugt verwendeten Zusammensetzungen (A) als Alkalisierungsmittel enthalten sein, bevorzugt in einer Gesamtmenge von 0,01 bis 10 Gew.-%, bevorzugt 0,1 bis 5 Gew.-%, besonders bevorzugt 0,5 bis 3 Gew.-%, jeweils bezogen auf das Gewicht der erfindungsgemäß verwendeten Zusammensetzung (A). Erfindungsgemäß besonders bevorzugt verwendete Zusammensetzungen (A) enthalten Mischungen der vorgenannten Alkalisierungsmittel, insbesondere Ammoniumhydroxid/Natriumsilikat/Kaliumhydroxid-Mischungen, Ammoniumhydroxid/Monoethanolamin/Natriumsilikat/Kaliumhydroxid-Mischungen, Monoethanolamin/ Natriumsilikat/Kaliumhydroxid-Mischungen, Ammoniumhydroxid/Natriumsilikat/Kaliumhydroxid/Arginin-Mischungen, Ammoniumhydroxid/Monoethanolamin/Natriumsilikat/Kaliumhydroxid/Arginin-Mischungen, Monoethanolamin/Natriumsilikat/Kaliumhydroxid/Arginin-Mischungen, Natriumsilikat/Magnesiumcarbonat-Mischungen und Natriummetasilikat/Magnesiumcarbonat-Mischungen.

### Konsistenzgeber

Erfindungsgemäß bevorzugt verwendete Zusammensetzungen (A), insbesondere die Zusammensetzungen (A-1), enthalten mindestens einen Konsistenzgeber. Geeignete Konsistenzgeber sind insbesondere Fettsubstanzen mit einem Schmelzpunkt von 25 °C oder darüber bei 1013 mbar Umgebungsdruck sowie polymere Verdicker, die von den erfindungsgemäß verwendeten Polysacchariden, die mit Calciumionen in wässrigem Medium ein Gel bilden, verschieden sind. Derartige Konsistenzgeber sind bevorzugt ausgewählt aus linearen gesättigten 1-Alkanolen mit 12 - 30 Kohlenstoffatomen und Glycerylfettsäureestern der allgemeinen Formel (I), 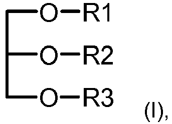 worin
R1, R2 und R3 unabhängig voneinander für ein Wasserstoffatom oder eine Gruppierung der Formel (II) stehen, 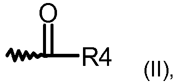 worin
R4 für eine unverzweigte oder verzweigte, gesättigte oder ungesättigte C₁₁-C₂₇-Alkylgruppe steht, mit der Maßgabe, dass mindestens einer und maximal zwei der Reste ausgewählt aus R1, R2 und R3 für eine Gruppierung der Formel (II) stehen.

Der Rest R4 in der Formel (II) steht für eine unverzweigte oder verzweigte, gesättigte oder ungesättigte C₁₁-C₂₇-Alkylgruppe.

Bevorzugt steht R4 für eine unverzweigte, gesättigte C₁₁-C₂₇-Alkylgruppe. Weiter bevorzugt steht R4 für eine unverzweigte, gesättigte C₁₃-C₂₃-Alkylgruppe. Besonders bevorzugt steht R4 für eine unverzweigte, gesättigte C₁₅-C₁₇-Alkylgruppe.

Erfindungsgemäß besonders bevorzugte Glycerylfettsäureester der allgemeinen Formel (I) sind ausgewählt aus mindestens einer Verbindung aus der Gruppe der Formeln (la) bis (Id):

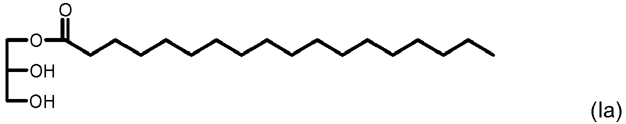

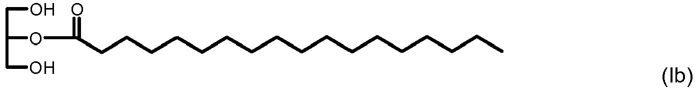

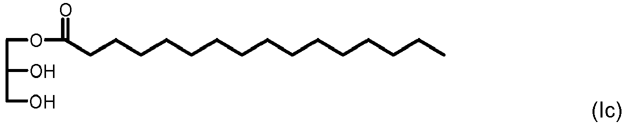

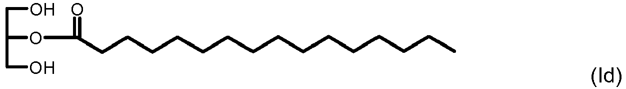

Die Verbindungen der Formeln (la) bis (Id) sind auch unter den Namen Glycerylmonostearat und Glycerylmonopalmitat bekannt.

Weitere erfindungsgemäß bevorzugt verwendete Blondier- oder Aufhellzusammensetzungen (A) sind dadurch gekennzeichnet, dass als Glycerylfettsäureester der allgemeinen Formel (I) mindestens eine Verbindung aus der Gruppe der Formeln (Ie) bis (Ih) enthalten ist:

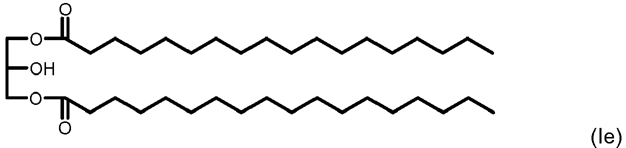

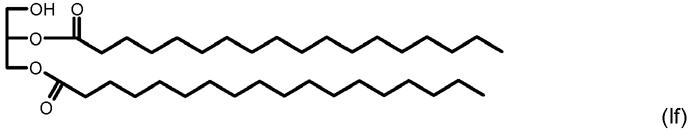

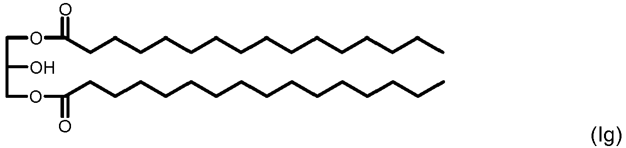

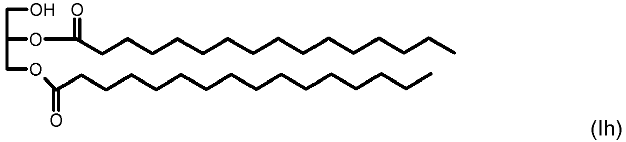

Die Verbindungen der Formel (Ie) bis (Ih) sind auch unter den Namen Glyceryldistearat und Glyceryldipalmitat bekannt.

Erfindungsgemäß bevorzugte lineare gesättigte 1-Alkanole mit 12 - 30 Kohlenstoffatomen sind ausgewählt aus Laurylalkohol, Myristylalkohol, Cetylalkohol, Stearylalkohol, Arachidylalkohol und Behenylalkohol sowie aus Mischungen dieser Alkanole, besonders bevorzugt aus Cetylalkohol, Stearylalkohol, Arachidylalkohol, Behenylalkohol und Cetylalkohol/ Stearylalkohol-Mischungen. Weitere erfindungsgemäß bevorzugte Konsistenzgeber sind Ethylenglycolmonosterat, Ethylenglycoldistearat, Wachse, Glycerintriester, die einen Schmelzpunkt von 25 °C oder darüber aufweisen, insbesondere hydriertes Rizinusöl (Castorwachs) und ethoxylierte hydrierte Rizinusöle, und Fettsäure-Fettalkoholester mit einem Schmelzpunkt von 25 °C oder darüber, sowie Mischungen dieser Substanzen.

Erfindungsgemäß bevorzugt verwendete Zusammensetzungen (A), insbesondere die Zusammensetzungen (A-1), enthalten mindestens einen Fettsubstanz-Konsistenzgeber in einer Gesamtmenge von 0,1 bis 30 Gew.-%, bevorzugt 0,5 bis 20 Gew.-%, besonders bevorzugt 2 bis 15 Gew.-%, außerordentlich bevorzugt 3 bis 10 Gew.-%, jeweils bezogen auf das Gewicht der Zusammensetzung (A). Weitere bevorzugte verwendete Zusammensetzungen (A), insbesondere die Zusammensetzungen (A-1), enthalten, jeweils bezogen auf ihr Gewicht, mindestens ein lineares gesättigtes 1-Alkanol mit 12 - 30 Kohlenstoffatomen in einer Gesamtmenge von 0,1 bis 30 Gew.-%, bevorzugt 0,5 bis 20 Gew.-%, besonders bevorzugt 2 bis 15 Gew.-%, außerordentlich bevorzugt 3 bis 10 Gew.-%, jeweils bezogen auf das Gewicht der Zusammensetzung (A).

In einer besonders bevorzugten Ausführungsform der Erfindung sind die erfindungsgemäß verwendeten Zusammensetzungen (A), (B), (C) und (D) frei von anionischen oder kationischen Assoziativpolymeren, die mit ggf. ethoxylierten, C₆ - C₂₄-Alkyl- oder C₆ - C₂₄-Alkenyl-Gruppen substituiert sind. In einer außerordentlich bevorzugten Ausführungsform der Erfindung sind die erfindungsgemäß verwendeten Zusammensetzungen (A), (B), (C) und (D) frei von anionischen oder kationischen Assoziativpolymeren, die mit ggf. ethoxylierten, C₆ - C₂₄-Alkyl- oder C₆ - C₂₄-AlkenylGruppen substituiert sind und ausgewählt sind aus Acrylates/Ceteth-20 Itaconate Copolymeren, Polyurethane-39-Polymeren, Acrylates/Beheneth-25 Methacrylate Copolymeren und Acrylates/C10-30 Alkyl Acrylate Crosspolymeren.

### Gelierendes Metallsalz in Zusammensetzung (A)

Sofern das mindestens eine Salz eines mehrwertigen Metallions, ausgewählt aus Calcium-, Strontium-, Barium- und Aluminiumsalzen mit einer Wasserlöslichkeit bei 20°C von mindestens 500 mg/l und unter bestimmten Bedingungen auch ausgewählt aus Kaliumsalzen mit einer Wasserlöslichkeit bei 20°C von mindestens 500 mg/l, in der Blondier- oder Aufhellzusammensetzung (A) enthalten ist, liegt es bevorzugt in einer Gesamtmenge von 0,1 - 5 Gew.-%, besonders bevorzugt 0,2 - 3 Gew.-%, außerordentlich bevorzugt 0,5 - 2,5 Gew.-%, jeweils bezogen auf das Gewicht der Zusammensetzung (A), vor. Besonders bevorzugt enthält die Blondier- oder Aufhellzusammensetzung (A) mindestens ein Salz d)i, das bei 20°C eine Wasserlöslichkeit von mindestens 500 mg/l, bevorzugt von mindestens 40 g/l, besonders bevorzugt von mindestens 200 g/l, außerordentlich bevorzugt von mindestens 350 g/l, aufweist. Das erfindungsgemäß außerordentlich bevorzugte Calciumchlorid hat eine Wasserlöslichkeit von 740 g/l bei 20°C. Andere erfindungsgemäß besonders bevorzugte Salze d)i sind ausgewählt aus Calciumacetat (Wasserlöslichkeit 374 g/l), Calciumlactat, Calciumgluconat, Calciumgluconatlactat. Ebenfalls bevorzugt sind Aluminiumchlorid, Aluminiumhydroxychlorid, Aluminiumsulfat, Aluminiumlactat, Aluminiumacetat, Aluminiumgluconat. Für die Gelierung von kappa-Carragheenan sind Kaliumchlorid, Kaliumacetat, Kaliumlactat, Kaliumgluconat und Kaliumsulfat bevorzugt, wobei Kaliumchlorid, Kaliumlactat, und Kaliumgluconat besonders bevorzugt sind. Geeignet sind weiterhin Strontiumchlorid und Bariumchlorid. Bevorzugt können auch Mischungen der vorgenannten Salze enthalten sein. Besonders bevorzugt enthält die Blondier- oder Aufhellzusammensetzung (A) mindestens ein Salz, ausgewählt aus Calciumchlorid, Calciumchlorid, Calciumlactat, Calciumgluconat und Calciumgluconatlactat sowie Mischungen hiervon in einer Gesamtmenge von 0,1 - 5 Gew.-%, besonders bevorzugt 0,2 - 3 Gew.-%, außerordentlich bevorzugt 0,5 - 2,5 Gew.-%, jeweils bezogen auf das Gewicht der Zusammensetzung (A). Außerordentlich bevorzugt enthält die Blondier- oder Aufhellzusammensetzung (A) 0,1 - 5 Gew.-%, besonders bevorzugt 0,2 - 3 Gew.-%, außerordentlich bevorzugt 0,5 - 2,5 Gew.-%, Calciumchlorid, jeweils bezogen auf das Gewicht der Zusammensetzung (A).

### Direktziehende Farbstoffe

In einer weiteren bevorzugten Ausführungsform enthalten die erfindungsgemäß verwendeten Zusammensetzungen (A) mindestens einen direktziehenden Farbstoff. Direktziehende Farbstoffe können in anionische, kationische und nichtionische direktziehende Farbstoffe unterteilt werden. Die direktziehenden Farbstoffe sind vorzugsweise ausgewählt aus den Nitrophenylendiaminen, den Nitroaminophenolen, den Azofarbstoffen, den Anthrachinonen, den Triarylmethanfarbstoffen oder den Indophenolen und deren physiologisch verträglichen Salzen. Bevorzugt ist mindestens ein direktziehender Farbstoff in einer Gesamtmenge von 0,001 bis 3 Gew.-%, bezogen auf das Gewicht der erfindungsgemäß verwendeten Zusammensetzung (A) enthalten. Direktziehende Farbstoffe dienen in oxidativen Blondiermitteln (A-1) und (A-2) zum Ausgleich unerwünschter Rot- und Orangetöne, die beim Abbau des haareigenen Melanins entstehen können.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen. Tetrabromphenolblau, Acid Red 33 und Acid Red 52 sowie deren Mischungen sind für Blondiermittel (A-1) und (A-2) erfindungsgemäß besonders bevorzugt.

Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, kationische Anthrachinonfarbstoffe, wie HC Blue 16 (Bluequat B) sowie direktziehende Farbstoffe, die einen Heterozyklus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 7, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

### Oxidationszusammensetzunq (B)

Der Abbau des haareigenen Farbstoffs Melanin zur Blondierung erfolgt erst durch den Einfluss einer Peroxidverbindung als Oxidationsmittel. Üblicherweise wird hierfür Wasserstoffperoxid verwendet. Der Einsatz von Wasserstoffperoxid kann nur in Form einer wässrigen Lösung erfolgen. Erfindungsgemäß bevorzugt verwendete Oxidationszusammensetzungen (B) sind dadurch gekennzeichnet, dass sie 0,5 bis 13 Gew.-%, weiter bevorzugt 1 bis 9 Gew.-%, besonders bevorzugt 2 bis 7 Gew.-% und ganz besonders bevorzugt 3 bis 4,5 Gew.-% Wasserstoffperoxid (berechnet als 100 %-iges H₂O₂) enthalten, jeweils bezogen auf das Gesamtgewicht der Oxidationszusammensetzung (B).

Blondiermittel (A-2), die besonders stark aufhellen sollen, zum Beispiel zur Blondierung oder Aufhellung von sehr dunklen, Melanin-reichen Keratinfasern, können darüber hinaus stark oxidierende Peroxidverbindungen, wie Kalium-, Natrium- und/oder Ammoniumpersulfat und/oder Natriumpercarbonat enthalten.

### Wasser

Die erfindungsgemäß verwendeten Oxidationszusammensetzungen (B) enthalten Wasser, und zwar bevorzugt in einer Menge von 20 bis 95 Gew.-%, bevorzugt 30 bis 90 Gew.-%, besonders bevorzugt 40 bis 85 Gew.-%, außerordentlich bevorzugt 50 bis 80 Gew.-%, jeweils bezogen auf das Gesamtgewicht der Oxidationszusammensetzung (B).

### pH-Wert

Erfindungsgemäß verwendete Oxidationszusammensetzungen (B) weisen einen pH-Wert im Bereich von 2,5 - 6,5, bevorzugt im Bereich von 3 - 5,5, besonders bevorzugt im Bereich von 3,5 bis 5,0, auf, jeweils bei 20°C gemessen.

### Gelbildendes Salz in der Oxidationszusammensetzung (B)

Sofern das mindestens eine Salz eines mehrwertigen Metallions, ausgewählt aus Calcium-, Strontium-, Barium- und Aluminiumsalzen mit einer Wasserlöslichkeit bei 20°C von mindestens 500 mg/l und unter bestimmten Bedingungen auch ausgewählt aus Kaliumsalzen mit einer Wasserlöslichkeit bei 20°C von mindestens 500 mg/l, in der Oxidationszusammensetzung (B) enthalten ist, liegt es bevorzugt in einer Gesamtmenge von 0,1 - 5 Gew.-%, besonders bevorzugt 0,2 - 3 Gew.-%, außerordentlich bevorzugt 0,5 - 2,5 Gew.-%, jeweils bezogen auf das Gewicht der Zusammensetzung (B), vor. Besonders bevorzugt enthält die Oxidationszusammensetzung (B) mindestens ein Salz d)i, das bei 20°C eine Wasserlöslichkeit von mindestens 500 mg/l, bevorzugt von mindestens 40 g/l, besonders bevorzugt von mindestens 200 g/l, außerordentlich bevorzugt von mindestens 350 g/l, aufweist. Das erfindungsgemäß außerordentlich bevorzugte Calciumchlorid hat eine Wasserlöslichkeit von 740 g/l bei 20°C. Andere erfindungsgemäß besonders bevorzugte Salze d)i sind ausgewählt aus Calciumacetat (Wasserlöslichkeit 374 g/l), Calciumlactat, Calciumgluconat, Calciumgluconatlactat. Ebenfalls bevorzugt sind Aluminiumchlorid, Aluminiumhydroxychlorid, Aluminiumsulfat, Aluminiumlactat, Aluminiumacetat, Aluminiumgluconat. Für die Gelierung von kappa-Carragheenan sind Kaliumchlorid, Kaliumacetat, Kaliumlactat, Kaliumgluconat und Kaliumsulfat bevorzugt, wobei Kaliumchlorid, Kaliumlactat, und Kaliumgluconat besonders bevorzugt sind. Geeignet sind weiterhin Strontiumchlorid und Bariumchlorid. Bevorzugt können auch Mischungen der vorgenannten Salze enthalten sein. Besonders bevorzugt enthält die Oxidationszusammensetzung (B) mindestens ein Salz, ausgewählt aus Calciumchlorid, Calciumchlorid, Calciumlactat, Calciumgluconat und Calciumgluconatlactat sowie Mischungen hiervon in einer Gesamtmenge von 0,1 - 5 Gew.-%, besonders bevorzugt 0,2 - 3 Gew.-%, außerordentlich bevorzugt 0,5 - 2,5 Gew.-%, jeweils bezogen auf das Gewicht der Zusammensetzung (B). Außerordentlich bevorzugt enthält die Oxidationszusammensetzung (B) 0,1 - 5 Gew.-%, besonders bevorzugt 0,2 - 3 Gew.-%, außerordentlich bevorzugt 0,5 - 2,5 Gew.-%, Calciumchlorid, jeweils bezogen auf das Gewicht der Zusammensetzung (B).

### Organische Lösemittel

Weiterhin können alle erfindungsgemäß verwendeten Zusammensetzungen, insbesondere die Oxidationszusammensetzungen (B) und die Blondier- oder Aufhellzusammensetzungen (A-1), weniger bevorzugt auch (A-2), mindestens ein organisches Lösemittel enthalten, das bevorzugt ausgewählt ist aus einem oder mehreren ein- oder mehrwertigen Alkoholen mit 2 bis 9 Kohlenstoffatomen, Polyethylenglycolen mit 2 bis 20 Ethylenglycoleinheiten sowie Mischungen dieser Lösemittel, bevorzugt in einer Gesamtmenge von 0,001 bis 80 Gew.-%, besonders bevorzugt 0,01 bis 50 Gew.-%, außerordentlich bevorzugt 0,5 bis 20 Gew.-%, weiter außerordentlich bevorzugt 1,5 bis 10 Gew.-%, jeweils bezogen auf das Gewicht der jeweiligen erfindungsgemäß verwendeten Zusammensetzung. Bevorzugte organische Lösemittel sind ausgewählt aus C₁-C₄-Alkoholen, insbesondere Ethanol und Isopropanol, weiterhin ausgewählt aus mehrwertigen Alkoholen mit 2 bis 9, bevorzugt ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,2-Hexandiol und 1,6-Hexandiol, Hexantriolen wie 1,2,6-Hexantriol, 2-Ethyl-2-hydroxymethyl-1,3-propandiol, Dipropylenglycol, Tripropylenglycol, Diglycerin und Triglycerin sowie weiterhin ausgewählt aus Polyethylenglycolen mit 2 bis 20 Ethylenglycoleinheiten, insbesondere PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind. Die Verwendung von Mischungen der vorgenannten Substanzen ist erfindungsgemäß ebenfalls bevorzugt.

### Mischungsverhältnisse (A) (B)

Weiterhin ist es für das Blondier- oder Aufhellergebnis wichtig, dass das anwendungsbereite Blondier- oder Aufhellmittel, das durch Vermischen einer erfindungsgemäß verwendeten Blondier-oder Aufhellzusammensetzung (A) mit einer erfindungsgemäß verwendeten Oxidationszusammensetzung (B) erhalten wird, einen pH-Wert im Bereich von 6,5 bis 11,0, bevorzugt 8 bis 10,5, besonders bevorzugt 8,5 bis 9,5, aufweist, jeweils gemessen bei 20°C. Bei diesen pH-Werten öffnet sich die äußere Keratinfaserschicht optimal zur Aufnahme des Oxidationsmittels und entfaltet sich die oxidative Wirkung des Wasserstoffperoxids und ggf. weiterer Peroxidverbindungen optimal. Weiterhin kann es erfindungsgemäß bevorzugt sein, das anwendungsbereite Blondier- oder Aufhellmittel durch Vermischen einer erfindungsgemäß verwendeten Aufhellzusammensetzung (A-1) mit einer erfindungsgemäß verwendeten Aufhellzusammensetzung (A-2) und einer erfindungsgemäß verwendeten Oxidationszusammensetzung (B) herzustellen.

Erfindungsgemäß bevorzugte Verfahren zur, bevorzugt multitonalen, Farbveränderung keratinischer Fasern sind daher dadurch gekennzeichnet, dass das Gewichtsverhältnis von Blondier- oder Aufhellzusammensetzung (A) zu Oxidationszusammensetzung (B) im Bereich von 1 : 10 bis 4:1, bevorzugt im Bereich von 1 : 5 bis 3 : 1, besonders bevorzugt im Bereich von 1 : 4 bis 2 : 1 und außerordentlich bevorzugt im Bereich von 1:2 bis 1:1 liegt.

Um die Mischbarkeit von (A) und (B) und die Auswaschbarkeit des Aufhell- oder Blondiermittels von den Keratinfasern nach Abschluss des erfindungsgemäßen Verfahrens zu begünstigen, ist es erfindungsgemäß bevorzugt, dass zumindest die Blondier- oder Aufhellzusammensetzung (A) mindestens ein Tensid enthält, bevorzugt in einer Gesamtmenge von 0,1 bis 15 Gew.-%, bevorzugt 1 bis 8 Gew.-%, bezogen auf das Gewicht der Zusammensetzung (A).

Auch die Zusammensetzung (B) enthält bevorzugt mindestens ein Tensid, bevorzugt in einer Gesamtmenge von 0,1 bis 10 Gew.-%, bevorzugt 1 bis 5 Gew.-%, bezogen auf das Gewicht der Zusammensetzung (B).

Geeignete Tenside, insbesondere für (A) und (B), sind nichtionische, anionische, kationische und amphotere Tenside. Bevorzugt sind nichtionische und anionische Tenside sowie Mischungen hiervon. Bevorzugte anionische Tenside sind Alkylsulfate, Alkylethersulfate und Ethercarbonsäuren mit 10 bis 18 C-Atomen in der Alkylgruppe und bis zu 12 Glykolethergruppen im Molekül. Besonders bevorzugte amphotere Tenside sind Cocamidopropylbetain, N-Kokosalkylaminopropionat, Kokosacylaminoethylaminopropionat und C₁₂-C₁₈-Acylsarcosin.

Bevorzugte nichtionische Tenside sind ausgewählt aus ethoxylierten C10-C24-Fettalkoholen mit einem Ethoxylierungsgrad von 4 bis 120, bevorzugt 10 bis 50, besonders bevorzugt 12 bis 20, wobei Mischungen dieser ethoxylierten C10-C24-Fettalkohole mit unterschiedlichen Ethoxylierungsgraden besonders bevorzugt sind. Weitere bevorzugte nichtionische Tenside sind ausgewählt aus ethoxylierten Glycerinmono-, -di- und -triestern von C10-C24-Fettsäuren, beispielsweise PEG-40 Castor Oil, PEG-40 Hydrogenated Castor Oil, PEG-60 Castor Oil oder PEG-60 Hydrogenated Castor Oil. Weitere bevorzugte nichtionische Tenside sind ausgewählt aus C₈ - C₂₂-Alkylmono- und - oligoglycosiden. C₈ -C₂₂-Alkylmono- und -oligoglycoside stellen bekannte, handelsübliche Tenside und Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, vorzugsweise 1 - 2, geeignet sind. Der Oligomerisierungsgrad ist dabei ein statistischer Mittelwert, dem eine für solche technischen Produkte übliche Homologenverteilung zugrunde liegt. Handelsübliche Produkte, die zum Beispiel unter dem Warenzeichen Plantacare® erhältlich sind, enthalten eine glucosidisch gebundene C₈-C₁₆-Alkylgruppe an einem Oligoglucosidrest, dessen mittlerer Oligomerisationsgrad bei 1 - 2, insbesondere 1,2 - 1,4, liegt. Besonders bevorzugte C₈ - C₂₂-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon.

Bevorzugte kationische Tenside sind beispielsweise Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen.

### Gelierzusammensetzung (C)

Um erfindungsgemäß die den Strähnchenfolien analog wirkenden Trennschichten oder eine Wasser- und Ammoniak-Barriereschicht aus Polysaccharidgel in situ auf den Keratinfasern herzustellen, wird auf die mit der Mischung (M) aus Blondier- oder Aufhellzusammensetzung (A) mit Oxidationszusammensetzung (B) behandelten Keratinfasern eine Gelierzusammensetzung (C) appliziert, beispielsweise mit einem Pinsel oder ähnlichen Applikator, oder - was besonders bevorzugt ist - durch Aufsprühen, wobei die Gelierzusammensetzung (C) enthält:
c)i. 0,1 - 3 Gew.-%, bevorzugt 0,2 - 2 Gew.-%, besonders bevorzugt 0,4 - 1 Gew.-%, bezogen auf das Gewicht der Zusammensetzung (C), mindestens eines Salzes eines mehrwertigen Metallions, ausgewählt aus Calcium-, Strontium-, Barium- und Aluminiumsalzen mit einer Wasserlöslichkeit bei 20°C von mindestens 500 mg/l, sowie für den Fall, dass das mit Calciumionen Gel bildende Polysaccharid kappa-Carragheenan oder eines seiner Salze umfasst, die Gelierzusammensetzung (C) 0,1 - 3 Gew.-%, bevorzugt 0,2 - 2 Gew.-%, besonders bevorzugt 0,4 - 1 Gew.-%, bezogen auf das Gewicht der Zusammensetzung (C), mindestens eines Salzes, ausgewählt aus Kalium-, Calcium-, Strontium-, Barium- und Aluminiumsalzen mit einer Wasserlöslichkeit bei 20°C von mindestens 500 mg/l enthält,
c)ii. 50 - 99 Gew.-%, bezogen auf das Gewicht der Zusammensetzung (C), Wasser,
c)iii. optional mindestens ein Kationpolymer, das bevorzugt ausgewählt ist aus kationisierten Guar-Ethern;
c)iv. wobei die Gelierzusammensetzung (C) einen pH-Wert im Bereich von 4 bis 12, bevorzugt im Bereich von 4 - 9, besonders bevorzugt 6,5 - 8, aufweist, gemessen bei 20°C.

### Gel bildendes Salz / Wasserlöslichkeit

Das mindestens eine Salz c)i weist bei 20°C eine Wasserlöslichkeit von mindestens 500 mg/l, bevorzugt von mindestens 40 g/l, besonders bevorzugt von mindestens 200 g/l, außerordentlich bevorzugt von mindestens 350 g/l, auf. Das erfindungsgemäß außerordentlich bevorzugte Calciumchlorid hat eine Wasserlöslichkeit von 740 g/l bei 20°C. Andere erfindungsgemäß besonders bevorzugte Salze c)i sind ausgewählt aus Calciumacetat (Wasserlöslichkeit 374 g/l), Calciumlactat, Calciumgluconat, Calciumgluconatlactat. Ebenfalls bevorzugt sind Aluminiumchlorid, Aluminiumhydroxychlorid, Aluminiumsulfat, Aluminiumlactat, Aluminiumacetat, Aluminiumgluconat. Für die Gelierung von kappa-Carragheenan sind Kaliumchlorid, Kaliumacetat, Kaliumlactat, Kaliumgluconat und Kaliumsulfat bevorzugt, wobei Kaliumchlorid, Kaliumlactat, und Kaliumgluconat besonders bevorzugt sind. Geeignet sind weiterhin Strontiumchlorid und Bariumchlorid. Bevorzugt können auch Mischungen der vorgenannten Salze enthalten sein.

Die Gelierzusammensetzung (C) enthält, jeweils bezogen auf ihr Gewicht, 50 - 99 Gew.-%, bevorzugt 70 - 95 Gew.-%, besonders bevorzugt 75 - 92 Gew.-% Wasser.

Die Gelierzusammensetzung (C) weist einen pH-Wert im Bereich von 4 bis 12, bevorzugt im Bereich von 4 - 9, besonders bevorzugt 6,5 - 8, auf, gemessen bei 20°C. Ob ein saurer oder ein alkalischer pH-Wert eingestellt ist, hängt unter anderem davon ab, ob die Viskosität der Zusammensetzung (C) zur Verbesserung ihrer Applikationseigenschaften, insbesondere zur Verbesserung des Sprühbildes, mit einem Verdickungsmittel angehoben werden soll. Zur bevorzugt moderaten Andickung der Zusammensetzung (C) sind besonders gut kationische Polymere geeignet. Bevorzugte kationische Polymere sind ausgewählt aus kationischen Guarderivaten, insbesondere aus kationisierten Guar-Ethern, insbesondere aus Polymeren mit den INCI-Bezeichnungen Hydroxypropyl Guar Hydroxypropyltrimonium Chloride und Guar Hydroxypropyltrimonium Chloride. Sofern die Gelierzusammensetzung (C) ein kationisches Polymer enthält, ist dieses in einer Gesamtmenge von 0,01 - 1 Gew.-%, bevorzugt 0,1 bis 0,8 Gew.-%, besonders bevorzugt 0,2 bis 0,5 Gew.-%, jeweils bezogen auf das Gewicht der Zusammensetzung (C), enthalten. Sofern die Gelierzusammensetzung (C) ein kationisches Polymer enthält, weist sie bevorzugt einen pH-Wert im Bereich von 4 - 6,5 auf, gemessen bei 20°C.

Die Einstellung des pH-Wertes erfolgt, wie auch für die übrigen erfindungsgemäß verwendeten Zusammensetzungen, mit üblichen pH-Stellmitteln, wie insbesondere Citronensäure, Milchsäure, NaOH, KOH und ähnlichen kosmetisch verträglichen Säuren und Basen.

In einer bevorzugten Ausführungsform des erfindungsgemäßen Aufhell- oder Blondierverfahrens wird die Gelierzusammensetzung (C) *in situ* oder erst 0,01 bis 24 Stunden vor Durchführung des erfindungsgemäßen Verfahrens durch Vermischen einer festen, bevorzugt pulverförmigen, Gelierzusammensetzung (C'), enthaltend das mindestens eine unter c)i genannte Salz in Pulverform und optional mindestens ein Kationpolymer, das bevorzugt ausgewählt ist aus kationisierten Guar-Ethern, in Pulverform, mit Wasser c) iii hergestellt. Besonders bevorzugt enthält die Gelierzusammensetzung (C') zusätzlich ein festes, bevorzugt pulverförmiges, pH-Stellmittel, z. B. eine Säure wie Citronensäure, und/oder ein Alkalisierungsmittel, wie Natriumsilikat.

### Polysaccharid-Zusammensetzung (D)

Eine besonders bevorzugte Ausführungsform des erfindungsgemäßen Aufhell- oder Blondierverfahrens ist dadurch gekennzeichnet, dass das mindestens eine Polysaccharid, das mit Calciumionen in wässrigem Medium ein Gel bilden kann und das bevorzugt ausgewählt ist aus Alginsäure, kappa-Carragheenan, iota-Carragheenan und Pektin, und/ oder mindestens einem Salz der vorgenannten Polysaccharide, das ausgewählt ist aus den Alkalimetall-, den Ammonium-, den Mono-, Di- und Trialkylammonium-, den Mono-, Di- und Trialkanolammonium- und den Magnesiumsalzen, wobei im Falle von Salzen des kappa-Carragheenans dessen Alkalimetall-Salz nur aus Lithium- und Natriumsalzen ausgewählt ist, bereitgestellt wird in Form einer Polysaccharid-Zusammensetzung (D), enthaltend
d)i. mindestens ein Polysaccharid, das mit Calciumionen in wässrigem Medium ein Gel bilden kann und ausgewählt ist aus Alginsäure, kappa-Carragheenan, iota-Carragheenan und Pektin, und/oder mindestens einem Salz der vorgenannten Polysaccharide, ausgewählt aus den Alkalimetall-, den Ammonium-, den Mono-, Di- und Trialkylammonium-, den Mono-, Di- und Trialkanolammonium- und den Magnesiumsalzen, bevorzugt in einer Gesamtmenge von 0,1 - 5 Gew.-%, bevorzugt 0,5 - 2 Gew.-%, besonders bevorzugt 0,5 - 2,5 Gew.-%, jeweils bezogen auf das Gewicht der Zusammensetzung (D), wobei im Falle von Salzen des kappa-Carragheenans dessen Alkalimetall-Salz nur aus Lithium- und Natriumsalzen ausgewählt ist,
d)ii. 50 - 99,9, bevorzugt 60 bis 95, besonders bevorzugt 80 bis 90 Gew.-% Wasser, jeweils bezogen auf das Gewicht der Zusammensetzung (D),
d)iii. optional mindestens ein Alkalisierungsmittel, das bevorzugt ausgewählt ist aus pulverförmigen Natriumsilikaten, insbesondere pulverförmigen Natriummetasilikaten,
d)iv. optional mindestens ein Polymer, enthaltend (Meth)Acrylsäure-, (Meth)Acrylsäureester-, (Meth)Acrylamid- und/oder 2-Acrylamido-2-methylpropansulfonsäure-Monomere, bevorzugt in einer Gesamtmenge von 0,05 - 3 Gew.-%, bevorzugt 0,2 - 0,5 Gew.-%, bezogen auf das Gewicht der Zusammensetzung (D),
d)v. optional mindestens ein Öl in einer Gesamtmenge von 0,1 bis 10 Gew.-%, bevorzugt 0,3 bis 5 Gew.-%, besonders bevorzugt 1 bis 3 Gew.-%, jeweils bezogen auf das Gewicht der Polysaccharid-Zusammensetzung (D),
wobei optional die Polysaccharid-Zusammensetzung (D) durch Vermischen einer festen, bevorzugt pulverförmigen, Polysaccharid-Zusammensetzung (D'), enthaltend d)i und optional d)iii und/oder optional d)iv, mit der Komponente d)ii und optional mit der Komponente d)v *in situ* oder 0,01 bis 24 Stunden vor Durchführung des erfindungsgemäßen Farbveränderungsverfahrens hergestellt wird,

In einer bevorzugten Ausführungsform des erfindungsgemäßen Aufhell- oder Blondierverfahrens wird die Polysaccharid-Zusammensetzung (D) *in situ* oder 0,01 bis 24 Stunden vor Durchführung des erfindungsgemäßen Farbveränderungsverfahrens durch Vermischen einer festen, bevorzugt pulverförmigen, Polysaccharid-Zusammensetzung (D'), enthaltend c)i und optional c)iii und/oder optional c)iv, mit der Komponente c)ii und optional mit der Komponente c)v hergestellt.

Bei der in situ Herstellung der Polysaccharid-Zusammensetzung (D) hat es sich bewährt, das mindestens eine Polysaccharid, das mit Calciumionen in wässrigem Medium ein Gel bilden kann, in 25°C bis 40 °C warmes Wasser aufzulösen, das mindestens ein Öl in einer Gesamtmenge von 0,1 bis 10 Gew.-%, bevorzugt 0,3 bis 5 Gew.-%, besonders bevorzugt 1 bis 3 Gew.-%, jeweils bezogen auf das Gewicht der Polysaccharid-Zusammensetzung (D), enthält. Das Öl sollte vor der Zugabe des Polysaccharids im Wasser enthalten sein. Dadurch wird die Auflösung des Polysaccharids deutlich begünstigt. Erfindungsgemäß außerordentlich bevorzugte Öle sind ausgewählt aus natürlichen und synthetischen Kohlenwasserstoffen, besonders bevorzugt aus Mineralölen, Paraffinölen, C₁₈-C₃₀-Isoparaffinen, insbesondere Isoeicosan, Polyisobutene und Polydecene, weiterhin ausgewählt aus C₈-C₁₆-Isoparaffinen, insbesondere aus Isodecan, Isododecan, Isotetradecan und Isohexadecan sowie Mischungen hiervon, sowie 1,3-Di-(2-ethylhexyl)-cyclohexan.

Weitere erfindungsgemäß bevorzugte Öle sind ausgewählt aus den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Dazu zählen Cetyl-2-ethylhexanoat, 2-Hexyldecylstearat (z. B. Eutanol® G 16 S), 2-Hexyldecyllaurat, Isodecylneopentanoat, Isononylisononanoat, 2-Ethylhexylpalmitat (z. B. Cegesoft® C 24) und 2-Ethylhexylstearat (z. B. Cetiol® 868). Ebenfalls bevorzugt sind Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und Ethylenglycoldipalmitat.

Weitere erfindungsgemäß bevorzugte Öle sind ausgewählt aus den Benzoesäureestern von linearen oder verzweigten C8-22-Alkanolen. Besonders bevorzugt sind Benzoesäure-C12-C15-alkylester, z. B. erhältlich als Handelsprodukt Finsolv® TN, Benzoesäureisostearylester, z. B. erhältlich als Handelsprodukt Finsolv® SB, Ethylhexylbenzoat, z. B. erhältlich als Handelsprodukt Finsolv® EB, und Benzoesäureoctyldocecylester, z. B. erhältlich als Handelsprodukt Finsolv® BOD.

Weitere erfindungsgemäß bevorzugte Öle sind ausgewählt aus Fettalkoholen mit 6 - 30 Kohlenstoffatomen, die ungesättigt oder verzweigt und gesättigt oder verzweigt und ungesättigt sind. Die verzweigten Alkohole werden häufig auch als Guerbet-Alkohole bezeichnet, da sie nach der Guerbet-Reaktion erhältlich sind. Bevorzugte Alkoholöle sind 2-Hexyldecanol (Eutanol® G 16), 2-Octyldodecanol (Eutanol® G), 2-Ethylhexylalkohol und Isostearylalkohol.

Weitere bevorzugte Öle sind ausgewählt aus Mischungen aus Guerbetalkoholen und Guerbetalkoholestern, z.B. dem Handelsprodukt Cetiol® PGL (2-Hexyldecanol und 2-Hexyldecyllaurat).

Weitere erfindungsgemäß bevorzugte kosmetische Öle sind ausgewählt aus den Triglyceriden (= Dreifachestern des Glycerins) von linearen oder verzweigten, gesättigten oder ungesättigten, gegebenenfalls hydroxylierten C8-30-Fettsäuren. Besonders bevorzugt kann die Verwendung natürlicher Öle, z.B. Amaranthsamenöl, Aprikosenkernöl, Arganöl, Avocadoöl, Babassuöl, Baumwollsaatöl, Borretschsamenöl, Camelinaöl, Distelöl, Erdnussöl, Granatapfelkernöl, Grapefruitsamenöl, Hanföl, Haselnussöl, Holundersamenöl, Johannesbeersamenöl, Jojobaöl, Leinöl, Macadamianussöl, Maiskeimöl, Mandelöl, Marulaöl, Nachtkerzenöl, Olivenöl, Palmöl, Palmkernöl, Paranussöl, Pekannussöl, Pfirsichkernöl Rapsöl, Rizinusöl, Sanddornfruchtfleischöl, Sanddornkernöl, Sesamöl, Sojaöl, Sonnenblumenöl, Traubenkernöl, Walnussöl, Wildrosenöl, Weizenkeimöl, und die flüssigen Anteile des Kokosöls und dergleichen sein. Bevorzugt sind aber auch synthetische Triglyceridöle, insbesondere Capric/ Caprylic Triglycerides, z. B. die Handelsprodukte Myritol® 318, Myritol® 331 (BASF) oder Miglyol® 812 (Hüls) mit unverzweigten Fettsäureresten sowie Glyceryltriisostearin mit verzweigten Fettsäureresten.

Weitere erfindungsgemäß besonders bevorzugte kosmetische Öle sind ausgewählt aus den Dicarbonsäureestern von linearen oder verzweigten C2-C10-Alkanolen, insbesondere Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weitere erfindungsgemäß bevorzugte kosmetische Öle sind ausgewählt aus den Anlagerungsprodukten von 1 bis 5 Propylenoxid-Einheiten an ein- oder mehrwertige C8-22-Alkanole, wie Octanol, Decanol, Decandiol, Laurylalkohol, Myristylalkohol und Stearylalkohol, z. B. PPG-2-Myristylether und PPG-3-Myristylether (Witconol® APM).

Weitere erfindungsgemäß bevorzugte kosmetische Öle sind ausgewählt aus den Anlagerungsprodukten von mindestens 6 Ethylenoxid- und/oder Propylenoxid-Einheiten an ein- oder mehrwertige C3-22-Alkanole wie Glycerin, Butanol, Butandiol, Myristylalkohol und Stearylalkohol, die gewünschtenfalls verestert sein können, z. B. PPG-14-Butylether (Ucon Fluid® AP), PPG-9-Butylether (Breox® B25), PPG-10-Butandiol (Macol® 57), PPG-15-Stearylether (Arlamol® E) und Glycereth-7-diisononanoat.

Weitere erfindungsgemäß bevorzugte kosmetische Öle sind ausgewählt aus den C8-C22-Fettalkoholestern einwertiger oder mehrwertiger C2-C7-Hydroxycarbonsäuren, insbesondere die Ester der Glycolsäure, Milchsäure, Äpfelsäure, Weinsäure, Citronensäure und Salicylsäure. Solche Ester auf Basis von linearen C14/15-Alkanolen, z. B. C12-C15-Alkyllactat, und von in 2-Position verzweigten C12/13-Alkanolen sind unter dem Warenzeichen Cosmacol® von der Firma Nordmann, Rassmann GmbH & Co, Hamburg, zu beziehen, insbesondere die Handelsprodukte Cosmacol® ESI, Cosmacol® EMI und Cosmacol® ETI.

Weitere erfindungsgemäß bevorzugte kosmetische Öle sind ausgewählt aus den symmetrischen, unsymmetrischen oder cyclischen Estern der Kohlensäure mit C₃₋₂₂-Alkanolen, C₃₋₂₂-Alkandiolen oder C₃₋₂₂-Alkantriolen, z. B. Dicaprylylcarbonat (Cetiol® CC) oder die Ester gemäß der Lehre der DE19756454 A1, insbesondere Glycerincarbonat.

Weitere kosmetische Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen.

Weitere kosmetische Öle, die erfindungsgemäß geeignet sind, sind ausgewählt aus den Siliconölen, zu denen z. B. Dialkyl- und Alkylarylsiloxane, wie beispielsweise Cyclopentasiloxan, Cyclohexasiloxan, Dimethylpolysiloxan und Methylphenylpolysiloxan, aber auch Hexamethyldisiloxan, Octamethyltrisiloxan und Decamethyltetrasiloxan zählen. Bevorzugt können flüchtige Siliconöle sein, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning enthalten sind. Ebenfalls geeignet sind flüchtige lineare Siliconöle, insbesondere Hexamethyldisiloxan (L₂), Octamethyltrisiloxan (L₃), Decamethyltetrasiloxan (L₄) sowie beliebige Zweier- und Dreiermischungen aus L₂, L₃ und/ oder L₄, bevorzugt solche Mischungen, wie sie z. B. in den Handelsprodukten DC 2-1184, Dow Corning® 200 (0,65 cSt) und Dow Corning® 200 (1,5 cSt) von Dow Corning enthalten sind. Bevorzugte nichtflüchtige Siliconöle sind ausgewählt aus höhermolekularen linearen Dimethylpolysiloxanen, im Handel erhältlich z. B. unter der Bezeichnung Dow Corning® 200 Fluid mit kinematischen Viskositäten (25°C) im Bereich von 5 - 100 cSt, bevorzugt 5 - 50 cSt oder auch 5 - 10 cSt, und Dimethylpolysiloxan mit einer kinematischen Viskosität (25°C) von etwa 350 cSt. Es kann erfindungsgemäß außerordentlich bevorzugt sein, Mischungen der vorgenannten Öle einzusetzen.

Auch durch Zugabe eines Tensids, zum Beispiel in einer Gesamtmenge von 0,05 bis 3 Gew.-%, bevorzugt 0,1 bis 1 Gew.-%, besonders bevorzugt 0,3 bis 0,8 Gew.-%, bezogen auf das Gewicht der Zusammensetzung (D), kann die Auflösung des Polysaccharids günstig beeinflusst werden. Allerdings kann ein Tensidgehalt bewirken, dass die Polysaccharid-Zusammensetzung (D), die zum Lösen des Polysaccharids geschüttelt werden sollte, zu stark schäumt, was ihre Applikation erschwert. Bevorzugt enthält die Polysaccharid-Zusammensetzung (D) daher kein Tensid.

Besonders bevorzugt verwendete Polysaccharid-Zusammensetzungen (D) und (D') sind dadurch gekennzeichnet, dass das mindestens eine Polysaccharid, das mit Calciumionen in wässrigem Medium ein Gel bilden kann, ausgewählt ist aus Alginsäure, Natriumalginat, Ammoniumalginat, Magnesiumalginat, Monoethanolammoniumalginat, kappa-Carragheenan, das Lithiumsalz von kappa-Carragheenan, das Natriumsalz von kappa-Carragheenan, iota-Carragheenan, das Lithiumsalz von iota-Carragheenan, das Natriumsalz von iota-Carragheenan, das Kaliumsalz von iota-Carragheenan, das Ammoniumsalz von iota-Carragheenan, Pektin, Natriumpektinat, Ammoniumpektinat, Kaliumpektinat und Magnesiumpektinat, sowie Mischungen dieser Substanzen. Erfindungsgemäß außerordentlich bevorzugt sind Mischungen aus Alginsäure und Natriumalginat, insbesondere Mischungen aus Alginsäure und Natriumalginat im Gewichtsverhältnis von Alginsäure zu Natriumalginat im Bereich von 1:2 bis 2:1, bevorzugt im Bereich von 0,8 bis 1,25, besonders bevorzugt im Gewichtsverhältnis 1:1.

Besonders bevorzugt enthält die Polysaccharid-Zusammensetzung (D) eine Mischung aus Alginsäure und Natriumalginat in einer Gesamtmenge von 0,1 - 5 Gew.-%, besonders bevorzugt 0,2 - 3 Gew.-%, außerordentlich bevorzugt 0,5 - 2,5 Gew.-%, jeweils bezogen auf das Gewicht der Zusammensetzung (D), wobei es bevorzugt ist, wenn das Gewichtsverhältnis von Alginsäure zu Natriumalginat im Bereich von 1:2 bis 2:1, bevorzugt im Bereich von 0,8 bis 1,25, besonders bevorzugt im Gewichtsverhältnis 1:1 liegt.

Es ist erfindungsgemäß bevorzugt, dass die Polysaccharid-Zusammensetzung (D) einen pH-Wert im Bereich von 7 bis 12, bevorzugt 8 bis 10, besonders bevorzugt 8,5 bis 9,5, aufweist, jeweils bei 20°C gemessen. Daher enthält die Polysaccharid-Zusammensetzung (D) bevorzugt mindestens ein Alkalisierungsmittel, das besonders bevorzugt ausgewählt ist aus pulverförmigen Natriumsilikaten, insbesondere pulverförmigen Natriummetasilikaten. Aber auch die anderen vorstehend aufgeführten Alkalisierungsmittel können zur Einstellung des pH-Werts der Polysaccharid-Zusammensetzung (D) eingesetzt werden. Für die Polysaccharid-Zusammensetzung (D') ist ein Gehalt an mindestens einem festen, bevorzugt pulverförmigen, Alkalisierungsmittel bevorzugt, das bevorzugt ausgewählt ist aus pulverförmigen Natriumsilikaten, insbesondere pulverförmigen Natriummetasilikaten.

Es kann erfindungsgemäß bevorzugt sein, dass die Polysaccharid-Zusammensetzung (D) oder die Polysaccharid-Zusammensetzung (D') mindestens eine Polymer, enthaltend (Meth)Acrylsäure-, (Meth)Acrylsäureester-, (Meth)Acrylamid- und/oder 2-Acrylamido-2-methylpropansulfonsäure-Monomere, enthält, bevorzugt in einer Gesamtmenge von 0,05 - 3 Gew.-%, bevorzugt 0,2 - 0,5 Gew.-%, bezogen auf das Gewicht der Zusammensetzung (D). Der Zusatz dieser Polymere dient vor allem dazu, die Löslichkeit des Polysaccharids bei der in situ-Herstellung der Zusammensetzung (D) zu verbessern. Bevorzugt ist das mindestens eine Polymer, enthaltend (Meth)Acrylsäure-, (Meth)Acrylsäureester-, (Meth)Acrylamid- und/oder 2-Acrylamido-2-methylpropansulfonsäure-Monomere, ausgewählt aus vernetzten Homopolymeren der Acrylsäure, vernetzten Homopolymeren der 2-Acrylamido-2-methylpropansulfonsäure, vernetzten Copolymeren bestehend aus Acrylsäure- und 2-Acrylamido-2-methylpropansulfonsäure-Einheiten, vernetzten Copolymeren bestehend aus 2-Methyl-2[(1-oxo-2-propenyl)amino]-1-propansulfonsäure-Einheiten und Hydroxyethylacrylat-Einheiten, vernetzten Copolymeren bestehend aus Acrylamid- und 2-Acrylamido-2-methylpropansulfonsäure-Einheiten, vernetzten Copolymeren bestehend aus Methacrylsäure-Einheiten und C1-C4-Alkylacrylat-Einheiten, vernetzten Copolymeren bestehend aus Acrylsäure-Einheiten und C1-C4-Alkylmethacrylat-Einheiten, sowie Mischungen hiervon.

Besonders bevorzugt weist das mindestens eine Polymer, enthaltend (Meth)Acrylsäure-, (Meth)Acrylsäureester-, (Meth)Acrylamid- und/oder 2-Acrylamido-2-methylpropansulfonsäure-Monomere, keine Monomere auf, die mit ggf. ethoxylierten, C₆ - C₂₄-Alkyl- oder C₆ - C₂₄-AlkenylGruppen substituiert sind.

Ein weiteres erfindungsgemäß bevorzugtes Aufhell- oder Blondierverfahren ist dadurch gekennzeichnet, dass es mit mindestens einer zweiten Blondier- oder Aufhellzusammensetzung (A'), (A"), (A'") usw. anstelle von (A) durchgeführt wird, wobei sich die Zusammensetzungen (A) und (A'), (A"), (A'") usw. zumindest in Bezug auf die enthaltenen direktziehenden Haarfarbstoffe qualitativ und/oder quantitativ voneinander unterscheiden

Für alle erfindungsgemäßen und erfindungsgemäß bevorzugten Ausführungsformen des vorliegenden Aufhell- oder Blondierverfahrens ist es weiterhin besonders bevorzugt, dass die Keratinfasersträhnen nicht mit festen flächigen Trennhilfen, insbesondere nicht mit Folien aus festen Materialien, wie Aluminium, Papier oder Styropor, versehen sind.

Ein weiterer Gegenstand der vorliegenden Erfindung ist ein Kit zur Aufhellung oder Blondierung keratinischer Fasern, umfassend die folgenden, physikalisch voneinander getrennten Komponenten (D) und (C) sowie optional (A) und optional (B):
I. eine Polysaccharid-Zusammensetzung (D), enthaltend
   d)i. mindestens ein Polysaccharid, das mit Calciumionen in wässrigem Medium ein Gel bilden kann und ausgewählt ist aus Alginsäure, kappa-Carragheenan, iota-Carragheenan und Pektin, und/oder mindestens einem Salz der vorgenannten Polysaccharide, ausgewählt aus den Alkalimetall-, den Ammonium-, den Mono-, Di- und Trialkylammonium-, den Mono-, Di- und Trialkanolammonium- und den Magnesiumsalzen, wobei im Falle von Salzen des kappa-Carragheenans dessen Alkalimetall-Salz nur aus Lithium- und Natriumsalzen ausgewählt ist, wobei Mischungen aus Alginsäure und Natriumalginat besonders bevorzugt sind,
   d)ii. mindestens ein Alkalisierungsmittel, das bevorzugt ausgewählt ist aus pulverförmigen Natriumsilikaten, insbesondere pulverförmigen Natriummetasilikaten,
   d)iii. optional mindestens ein Polymer, enthaltend (Meth)Acrylsäure-, (Meth)Acrylsäureester-, (Meth)Acrylamid- und/oder 2-Acrylamido-2-methylpropansulfonsäure-Monomere, das bevorzugt keine Monomere aufweist, die mit ggf. ethoxylierten, C₆ - C₂₄-Alkyl- oder C₆ - C₂₄-Alkenyl-Gruppen substituiert sind;
II. eine Gelierzusammensetzung (C'), enthaltend
   c)i. mindestens ein Salz eines mehrwertigen Metallions, ausgewählt aus Calcium-, Strontium-, Barium- und Aluminiumsalzen mit einer Wasserlöslichkeit bei 20°C von mindestens 500 mg/l, sowie für den Fall, dass das mit Calciumionen Gel bildende Polysaccharid kappa-Carragheenan oder eines seiner Salze umfasst, mindestens ein Salz, ausgewählt aus Kalium-, Calcium-, Strontium-, Barium- und Aluminiumsalzen mit einer Wasserlöslichkeit bei 20°C von mindestens 500 mg/l;
   c)ii. optional mindestens ein Kationpolymer, das bevorzugt ausgewählt ist aus kationisierten Guar-Ethern,
III. optional eine Blondier- oder Aufhellzusammensetzung (A), enthaltend
   a)i mindestens ein Alkalisierungsmittel,
   a)ii optional mindestens einen Konsistenzgeber und
   a)iii optional mindestens ein Lösemittel, ausgewählt aus Wasser, einem oder mehreren ein- oder mehrwertigen Alkoholen mit 2 bis 9 Kohlenstoffatomen sowie Mischungen dieser Lösemittel und
   a)iv optional mindestens einen direktziehenden Farbstoff,
   wobei die Blondier- oder Aufhellzusammensetzung (A), sofern sie, bezogen auf ihr Gewicht, mehr als 7 Gew.-% Wasser enthält, einen pH-Wert im Bereich von 6,0 bis 11,5 aufweist, gemessen bei 20°C, und sofern die Zusammensetzung (A), bezogen auf ihr Gewicht, 0 bis 7 Gew.-% Wasser enthält, ihre 30 Gew.-%ige Dispersion in Wasser einen pH-Wert im Bereich von 6,0 bis 12,0, bevorzugt 7,5 - 11,5, besonders bevorzugt 8 bis 11,0, aufweist, jeweils gemessen bei 20°C;
IV. optional eine Oxidationszusammensetzung (B), enthaltend
   b)i. 1 - 18 Gew.-% Wasserstoffperoxid, bezogen auf das Gewicht der Zusammensetzung (B), und
   b)ii. Wasser,
   b)iii. wobei die Oxidationszusammensetzung (B) einen pH-Wert im Bereich von einen pH-Wert im Bereich von 2,5 - 6,5, bevorzugt im Bereich von 3 - 5,5, besonders bevorzugt im Bereich von 3,5 bis 5,0, aufweist, jeweils bei 20°C gemessen.

Für erfindungsgemäße und erfindungsgemäß bevorzugte Kits gilt mutatis mutandis das vorstehend zu den erfindungsgemäßen und erfindungsgemäß bevorzugten Aufhell- und Blondierverfahren. Erfindungsgemäße und erfindungsgemäß bevorzugte Kits haben den Vorteil, dass die wesentlichen Inhaltsstoffe der Zusammensetzungen (C) und (D) in platz- und verpackungssparender Weise, beispielsweise in Sachets, die bevorzugt eine wasserdampfundurchlässige Schicht aufweisen, kommerzialisiert werden können. So kann beispielsweise der Friseur durch einfaches Vermischen mit der Zusammensetzungen (C') und (D') mit Wasser und ggf. einer öligen Haarpflegezusammensetzung die Zusammensetzungen (C) und (D) selbst herstellen. Bevorzugt umfasst das Kit auch eine Gebrauchsanweisung für die Durchführung erfindungsgemäßer und erfindungsgemäß bevorzugter Aufhell- und Blondierverfahren.

Ein weiterer Gegenstand der vorliegenden Erfindung ist die Verwendung von mindestens einem Polysaccharidsalz, ausgewählt aus Calciumalginat, Kalium-kappa-Carragheenat, Calcium-kappa-Carragheenat, Calcium-iota-Carragheenat, Calciumpektinat, Aluminiumalginat, Aluminium-kappa-Carragheenat, Aluminium-iota-Carragheenat, Aluminiumpektinat, Strontiumalginat, Strontiumkappa-Carragheenat, Strontium-iota-Carragheenat, Strontiumpektinat, Bariumalginat, Bariumkappa-Carragheenat, Barium-iota-Carragheenat, Bariumpektinat, sowie Mischungen dieser Polysaccharidsalze, zur Beschichtung von Keratinfaserpartien oder Keratinfasersträhnen in einem Verfahren zur Aufhellung oder Blondierung keratinischer Fasern, wobei das Aufhell- und Blondierverfahren bevorzugt ein Verfahren gemäß einem der Ansprüche 1 - 10 oder 12 ist.

Für diese erfindungsgemäße Verwendung und bevorzugte Ausführungsformen gilt mutatis mutandis das vorstehend zu den erfindungsgemäßen und erfindungsgemäß bevorzugten Aufhell- und Blondierverfahren und Kits.

Ein weiterer Gegenstand der vorliegenden Erfindung ist die Verwendung von mindestens einem Polysaccharidsalz, ausgewählt aus Calciumalginat, Kalium-kappa-Carragheenat, Calcium-kappa-Carragheenat, Calcium-iota-Carragheenat, Calciumpektinat, Aluminiumalginat, Aluminium-kappa-Carragheenat, Aluminium-iota-Carragheenat, Aluminiumpektinat, Strontiumalginat, Strontiumkappa-Carragheenat, Strontium-iota-Carragheenat, Strontiumpektinat, Bariumalginat, Bariumkappa-Carragheenat, Barium-iota-Carragheenat, Bariumpektinat, sowie Mischungen dieser Polysaccharidsalze, in einem Verfahren zur Aufhellung und/oder Blondierung keratinischer Fasern in Gegenwart von Ammoniumhydroxid oder einem Ammoniumsalz, wobei das Aufhell- und/oder Blondierverfahren bevorzugt ein Verfahren gemäß einem der Ansprüche 1 - 10 oder 12 ist, zur Reduzierung der Ammoniakfreisetzung und/oder zur Reduzierung der Antrocknens der Aufhell- und/oder Blondierzusammensetzung.

Für diese erfindungsgemäße Verwendung und bevorzugte Ausführungsformen gilt mutatis mutandis das vorstehend zu den erfindungsgemäßen und erfindungsgemäß bevorzugten Aufhell- und Blondierverfahren und Kits.

## Patentansprüche

1. Verfahren zur Aufhellung und/oder Blondierung keratinischer Fasern, **gekennzeichnet durch** folgende Verfahrensschritte:
a. Bereitstellen einer Blondier- oder Aufhellzusammensetzung (A), enthaltend
a)i. mindestens ein Alkalisierungsmittel,
a)ii. optional mindestens einen Konsistenzgeber und
a)iii. optional mindestens ein Lösemittel, ausgewählt aus Wasser und mindestens einem organischen Lösemittel, das bevorzugt ausgewählt ist aus einem oder mehreren ein- oder mehrwertigen Alkoholen mit 2 bis 9 Kohlenstoffatomen, Polyethylenglycolen mit 2 bis 20 Ethylenglycoleinheiten sowie Mischungen dieser Lösemittel, und
a)iv. optional mindestens einen direktziehenden Haarfarbstoff,
a)v. wobei die Blondier- oder Aufhellzusammensetzung (A), sofern sie, bezogen auf ihr Gewicht, mehr als 7 Gew.-% Wasser enthält, einen pH-Wert im Bereich von 6,0 bis 11,5 aufweist, gemessen bei 20°C, und sofern die Zusammensetzung (A), bezogen auf ihr Gewicht, 0 bis 7 Gew.-% Wasser enthält, ihre 30 Gew.-%ige Dispersion in Wasser einen pH-Wert im Bereich von 6,0 bis 12,0, bevorzugt 7,5 -11,5, besonders bevorzugt 8 bis 11,0, aufweist, jeweils gemessen bei 20°C;
b. Bereitstellen einer Oxidationszusammensetzung (B), enthaltend
b)i. 1 - 18 Gew.-% Wasserstoffperoxid, bezogen auf das Gewicht der Zusammensetzung (B), und
b)ii. Wasser,
b)iii. wobei die Oxidationszusammensetzung (B) einen pH-Wert im Bereich von einen pH-Wert im Bereich von 2,5 - 6,5, bevorzugt im Bereich von 3 - 5,5, besonders bevorzugt im Bereich von 3,5 bis 5,0, aufweist, jeweils bei 20°C gemessen;
c. Bereitstellen einer Polysaccharid-Zusammensetzung (D), enthaltend
c)i. mindestens ein Polysaccharid, das mit Calciumionen in wässrigem Medium ein Gel bilden kann und ausgewählt ist aus Alginsäure, kappa-Carragheenan, iota-Carragheenan und Pektin, und/oder mindestens einem Salz der vorgenannten Polysaccharide, ausgewählt aus den Alkalimetall-, den Ammonium-, den Mono-, Di- und Trialkylammonium-, den Mono-, Di- und Trialkanolammonium- und den Magnesiumsalzen, bevorzugt in einer Gesamtmenge von 0,1 - 5 Gew.-%, bevorzugt 0,5 - 2 Gew.-%, besonders bevorzugt 0,5 - 2,5 Gew.-%, jeweils bezogen auf das Gewicht der Zusammensetzung (D), wobei im Falle von Salzen des kappa-Carragheenans dessen Alkalimetall-Salz nur aus Lithium- und Natriumsalzen ausgewählt ist,
c)ii. 50 - 99,9, bevorzugt 60 bis 95, besonders bevorzugt 80 bis 90 Gew.-% Wasser, jeweils bezogen auf das Gewicht der Zusammensetzung (D),
c)iii. optional mindestens ein Alkalisierungsmittel, das bevorzugt ausgewählt ist aus pulverförmigen Natriumsilikaten, insbesondere pulverförmigen Natriummetasilikaten,
c)iv. optional mindestens ein Polymer, enthaltend (Meth)Acrylsäure-, (Meth)Acrylsäureester-, (Meth)Acrylamid- und/oder 2-Acrylamido-2-methylpropansulfonsäure-Manomere, bevorzugt in einer Gesamtmenge von 0,05 - 3 Gew.-%, bevorzugt 0,2 - 0,5 Gew.-%, bezogen auf das Gewicht der Zusammensetzung (D),
c)v. optional mindestens ein Öl in einer Gesamtmenge von 0,1 bis 10 Gew.-%, bevorzugt 0,3 bis 5 Gew.-%, besonders bevorzugt 1 bis 3 Gew.-%, jeweils bezogen auf das Gewicht der Polysaccharid-Zusammensetzung (D),
wobei optional die Polysaccharid-Zusammensetzung (D) durch Vermischen einer festen, bevorzugt pulverförmigen, Polysaccharid-Zusammensetzung (D'), enthaltend c)i und optional c)iii und/oder optional c)iv, mit der Komponente c)ii und optional mit der Komponente c)v *in situ* oder 0,01 bis 24 Stunden vor Durchführung des erfindungsgemäßen Farbveränderungsverfahrens hergestellt wird,
anschließend, bevorzugt 1 bis 600 Sekunden danach,
d. Herstellen einer Mischung (M) aus (A) und (B), die einen pH-Wert im Bereich von 6,0 bis 11 aufweist, jeweils gemessen bei 20°C; nach 1 bis 600 Sekunden dann
e. Applikation zumindest einer Teilmenge von (M) auf mindestens eine zu blondierende und/oder aufzuhellende Keratinfaserpartie oder Applikation des Mittels (M) auf das gesamte zu behandelnde Keratinfaserensemble;
1 bis 600 Sekunden, bevorzugt 5 bis 300 Sekunden, besonders bevorzugt 5 bis 60 Sekunden danach
f. Applizieren, bevorzugt Aufsprühen, der Polysaccharid-Zusammensetzung (D), auf die mit (M) beaufschlagte(n) Keratinfaserpartie(n) oder das mit (M) beaufschlagte Keratinfaserensemble;
g. ggf. Wiederholen der Schritte e. und f. im gewünschten Umfang,
h. für eine Zeit von 0,1 bis 60 Minuten, bevorzugt 1 bis 50 Minuten, besonders bevorzugt 10 bis 45 Minuten, außerordentlich bevorzugt 30 bis 45 Minuten Belassen auf den keratinischen Fasern, anschließend Spülen der keratinischen Fasern mit Wasser und ggf. einem Reinigungsmittel, ggf. Nachbehandlung der Fasern mit einem Konditioniermittel und anschließendes Trocknen,
**dadurch gekennzeichnet, dass** das Verfahren die Applikation von mindestens einem Salz eines mehrwertigen Metallions, ausgewählt aus Calcium-, Strontium-, Barium- und Aluminiumsalzen mit einer Wasserlöslichkeit bei 20°C von mindestens 500 mg/l, umfasst, sowie für den Fall, dass das mit Calciumionen Gel bildende Polysaccharid kappa-Carragheenan oder eines seiner Salze umfasst, das Verfahren die Applikation von mindestens einem Salz, ausgewählt aus Kalium-, Calcium-, Strontium-, Barium- und Aluminiumsalzen mit einer Wasserlöslichkeit bei 20°C von mindestens 500 mg/l enthält umfasst,
wobei diese Salze nicht in der Polysaccharid-Zusammensetzung (D) enthalten sind.

2. Verfahren gemäß Anspruch 1, **dadurch gekennzeichnet, dass** die Blondier- oder Aufhellzusammensetzung (A) 0,1 - 3 Gew.-%, bevorzugt 0,2 - 2 Gew.-%, besonders bevorzugt 0,4 - 1 Gew.-%, bezogen auf das Gewicht der Zusammensetzung (A), mindestens eines Salzes eines mehrwertigen Metallions, ausgewählt aus Calcium-, Strontium-, Barium- und Aluminiumsalzen mit einer Wasserlöslichkeit bei 20°C von mindestens 500 mg/l, enthalt, sowie für den Fall, dass das mit Calciumionen Gel bildende Polysaccharid kappa-Carragheenan oder eines seiner Salze umfasst, die Blondier- oder Aufhellzusammensetzung (A) 0,1 - 3 Gew.-%, bevorzugt 0,2 - 2 Gew.-%, besonders bevorzugt 0,4 - 1 Gew.-%, bezogen auf das Gewicht der Zusammensetzung (A), mindestens eines Salzes, ausgewählt aus Kalium-, Calcium-, Strontium-, Barium- und Aluminiumsalzen mit einer Wasserlöslichkeit bei 20°C von mindestens 500 mg/l enthält.

3. Verfahren gemäß Anspruch 1 oder 2, **dadurch gekennzeichnet, dass** die Oxidationszusammensetzung (B) 0,1 - 3 Gew.-%, bevorzugt 0,2 - 2 Gew.-%, besonders bevorzugt 0,4 - 1 Gew.-%, bezogen auf das Gewicht der Zusammensetzung (B), mindestens eines Salzes eines mehrwertigen Metallions, ausgewählt aus Calcium-, Strontium-, Barium- und Aluminiumsalzen mit einer Wasserlöslichkeit bei 20°C von mindestens 500 mg/l, enthält, sowie für den Fall, dass das mit Calciumionen Gel bildende Polysaccharid kappa-Carragheenan oder eines seiner Salze umfasst, die Oxidationszusammensetzung (B) 0,1 - 3 Gew.-%, bevorzugt 0,2 - 2 Gew.-%, besonders bevorzugt 0,4 - 1 Gew.-%, bezogen auf das Gewicht der Zusammensetzung (B), mindestens eines Salzes, ausgewählt aus Kalium-, Calcium-, Strontium-, Barium- und Aluminiumsalzen mit einer Wasserlöslichkeit bei 20°C von mindestens 500 mg/l enthält.

4. Verfahren gemäß einem der Ansprüche 1 - 3, **dadurch gekennzeichnet, dass** das mindestens eine Salz eines mehrwertigen Metallions, ausgewählt aus Calcium-, Strontium-, Barium- und Aluminiumsalzen mit einer Wasserlöslichkeit bei 20°C von mindestens 500 mg/l, sowie für den Fall, dass das mit Calciumionen Gel bildende Polysaccharid kappa-Carragheenan oder eines seiner Salze umfasst, das mindestens eine Salz eines mehrwertigen Metallions, ausgewählt aus Kalium-, Calcium-, Strontium-, Barium- und Aluminiumsalzen mit einer Wasserlöslichkeit bei 20°C von mindestens 500 mg/l, bereitgestellt wird in Form einer Gelierzusammensetzung (C), enthaltend
d)i. 0,1 - 3 Gew.-%, bevorzugt 0,2 - 2 Gew.-%, besonders bevorzugt 0,4 - 1 Gew.-%, bezogen auf das Gewicht der Zusammensetzung (C), mindestens eines Salzes eines mehrwertigen Metallions, ausgewählt aus Calcium-, Strontium-, Barium- und Aluminiumsalzen mit einer Wasserlöslichkeit bei 20°C von mindestens 500 mg/l, sowie für den Fall, dass das mit Calciumionen Gel bildende Polysaccharid kappa-Carragheenan oder eines seiner Salze umfasst, die Gelierzusammensetzung (C) 0,1 - 3 Gew.-%, bevorzugt 0,2 - 2 Gew.-%, besonders bevorzugt 0,4 - 1 Gew.-%, bezogen auf das Gewicht der Zusammensetzung (C), mindestens eines Salzes, ausgewählt aus Kalium-, Calcium-, Strontium-, Barium- und Aluminiumsalzen mit einer Wasserlöslichkeit bei 20°C von mindestens 500 mg/l enthält,
d)ii. 50 - 99 Gew.-%, bezogen auf das Gewicht der Zusammensetzung (C), Wasser,
d)iii. optional mindestens ein Kationpolymer, das bevorzugt ausgewählt ist aus kationisierten Guar-Ethern;
d)iv. wobei die Gelierzusammensetzung (C) einen pH-Wert im Bereich von 4 bis 12, bevorzugt im Bereich von 4 - 9, besonders bevorzugt 6,5 - 8, aufweist, gemessen bei 20°C;
wobei optional die Gelierzusammensetzung (C) *in situ* oder 0,01 bis 24 Stunden vor Durchführung des erfindungsgemäßen Blondier- oder Aufhellverfahrens durch Vermischen einer festen, bevorzugt pulverförmigen, Gelierzusammensetzung (C'), enthaltend das mindestens eine unter d)i genannte Salz in Pulverform und optional mindestens ein Kationpolymer, das bevorzugt ausgewählt ist aus kationisierten Guar-Ethern, in Pulverform, mit Wasser d) iii hergestellt wird,
**dadurch gekennzeichnet, dass** die Gelierzusammensetzung (C) in einem Verfahrensschritt
- nach dem Applikationsschritt e und vor dem Applizieren, bevorzugt Aufsprühen, der Polysaccharid-Zusammensetzung (D) auf die mit der Mischung (M) aus (A) und (B) beaufschlagte(n) keratinische(n) Faser(n) appliziert, bevorzugt aufgesprüht, wird, oder
- vor dem Applikationsschritt e zu einer der Zusammensetzungen (A) oder (B) oder zur Mischung (M) aus (A) und (B) gegeben wird oder
- nach dem Applizieren, bevorzugt Aufsprühen, der Polysaccharid-Zusammensetzung (D) auf die mit der Mischung (M) aus (A) und (B) beaufschlagte(n) keratinische(n) Faser(n) appliziert, bevorzugt aufgesprüht, wird.

5. Verfahren gemäß einem der Ansprüche 1 - 4, **dadurch gekennzeichnet, dass** das Gewichtsverhältnis von Blondier- oder Aufhellzusammensetzung (A) zu Oxidationszusammensetzung (B) im Bereich von 1 : 10 bis 4:1, bevorzugt im Bereich von 1 : 5 bis 3 : 1, besonders bevorzugt im Bereich von 1 : 4 bis 2 : 1 und außerordentlich bevorzugt im Bereich von 1:2 bis 1:1 liegt.

6. Verfahren gemäß Anspruch 4 oder 5, **dadurch gekennzeichnet, dass** das Gewichtsverhältnis von Gelierzusammensetzung (C) zur Mischung (M) aus Blondier- oder Aufhellzusammensetzung (A) und Oxidationszusammensetzung (B) im Bereich von 1 : 20 bis 1:2, bevorzugt im Bereich von 1:10 bis 1:5 liegt.

7. Verfahren gemäß einem der Ansprüche 1 - 6, **dadurch gekennzeichnet, dass** das Gewichtsverhältnis von Polysaccharid-Zusammensetzung (D) zur Mischung (M) aus Blondier- oder Aufhellzusammensetzung (A) und Oxidationszusammensetzung (B) im Bereich von 1 : 20 bis 1:2, bevorzugt im Bereich von 1:10 bis 1:5 liegt.

8. Verfahren gemäß einem der Ansprüche 4 - 7, **dadurch gekennzeichnet, dass** das Gewichtsverhältnis von Polysaccharid-Zusammensetzung (D) zur Gelierzusammensetzung (C) im Bereich von 0,2 : 1 bis 2:1, bevorzugt im Bereich von 0,5 : 1 bis 1,5 : 1 liegt und besonders bevorzugt 1:1 beträgt.

9. Verfahren gemäß einem der Ansprüche 1 - 8, **dadurch gekennzeichnet, dass** es mit mindestens einer zweiten Blondier- oder Aufhellzusammensetzung (A'), (A"), (A"') usw. anstelle von (A) durchgeführt wird, wobei sich die Zusammensetzungen (A) und (A'), (A"), (A'") usw. zumindest in Bezug auf die enthaltenen direktziehenden Haarfarbstoffe qualitativ und/oder quantitativ voneinander unterscheiden.

10. Verfahren gemäß einem der Ansprüche 1 - 9, **dadurch gekennzeichnet, dass** die Keratinfasersträhnen nicht mit festen flächigen Trennhilfen, insbesondere nicht mit Folien aus festen Materialien, wie Aluminium, Papier oder Styropor, versehen sind.

11. Kit zur Aufhellung und/oder Blondierung keratinischer Fasern, umfassend die folgenden, physikalisch voneinander getrennten Komponenten (D) und (C) sowie optional (A) und optional (B):
I. eine Polysaccharid-Zusammensetzung (D), enthaltend
d)i. mindestens ein Polysaccharid, das mit Calciumionen in wässrigem Medium ein Gel bilden kann und ausgewählt ist aus Alginsäure, kappa-Carragheenan, iota-Carragheenan und Pektin, und/oder mindestens einem Salz der vorgenannten Polysaccharide, ausgewählt aus den Alkalimetall-, den Ammonium-, den Mono-, Di- und Trialkylammonium-, den Mono-, Di- und Trialkanolammonium- und den Magnesiumsalzen, wobei im Falle von Salzen des kappa-Carragheenans dessen Alkalimetall-Salz nur aus Lithium- und Natriumsalzen ausgewählt ist, wobei Mischungen aus Alginsäure und Natriumalginat besonders bevorzugt sind,
d)ii. mindestens ein Alkalisierungsmittel, das bevorzugt ausgewählt ist aus pulverförmigen Natriumsilikaten, insbesondere pulverförmigen Natriummetasilikaten,
d)iii. optional mindestens ein Polymer, enthaltend (Meth)Acrylsäure-, (Meth)Acrylsäureester-, (Meth)Acrylamid- und/oder 2-Acrylamido-2-methylpropansulfonsäure-Monomere, das bevorzugt keine Monomere aufweist, die mit ggf. ethoxylierten, C₆ - C₂₄-Alkyl- oder C₆ - C₂₄-Alkenyl-Gruppen substituiert sind;
II. eine Gelierzusammensetzung (C'), enthaltend
c)i. mindestens ein Salz eines mehrwertigen Metallions, ausgewählt aus Calcium-, Strontium-, Barium- und Aluminiumsalzen mit einer Wasserlöslichkeit bei 20°C von mindestens 500 mg/l, sowie für den Fall, dass das mit Calciumionen Gel bildende Polysaccharid kappa-Carragheenan oder eines seiner Salze umfasst, mindestens ein Salz, ausgewählt aus Kalium-, Calcium-, Strontium-, Barium- und Aluminiumsalzen mit einer Wasserlöslichkeit bei 20°C von mindestens 500 mg/l;
c)ii. optional mindestens ein Kationpolymer, das bevorzugt ausgewählt ist aus kationisierten Guar-Ethern,
III. optional eine Blondier- oder Aufhellzusammensetzung (A), enthaltend
a)i mindestens ein Alkalisierungsmittel,
a)ii optional mindestens einen Konsistenzgeber und
a)iii optional mindestens ein Lösemittel, ausgewählt aus Wasser, einem oder mehreren ein- oder mehrwertigen Alkoholen mit 2 bis 9 Kohlenstoffatomen sowie Mischungen dieser Lösemittel und
a)iv optional mindestens einen direktziehenden Haarfarbstoff,
wobei die Blondier- oder Aufhellzusammensetzung (A), sofern sie, bezogen auf ihr Gewicht, mehr als 7 Gew.-% Wasser enthält, einen pH-Wert im Bereich von 6,0 bis 11,5 aufweist, gemessen bei 20°C, und sofern die Zusammensetzung (A), bezogen auf ihr Gewicht, 0 bis 7 Gew.-% Wasser enthält, ihre 30 Gew.-%ige Dispersion in Wasser einen pH-Wert im Bereich von 6,0 bis 12,0, bevorzugt 7,5 - 11,5, besonders bevorzugt 8 bis 11,0, aufweist, jeweils gemessen bei 20°C;
IV. optional eine Oxidationszusammensetzung (B), enthaltend
b)i. 1 - 18 Gew.-% Wasserstoffperoxid, bezogen auf das Gewicht der Zusammensetzung (B), und
b)ii. Wasser,
b)iii. wobei die Oxidationszusammensetzung (B) einen pH-Wert im Bereich von einen pH-Wert im Bereich von 2,5 - 6,5, bevorzugt im Bereich von 3 - 5,5, besonders bevorzugt im Bereich von 3,5 bis 5,0, aufweist, jeweils bei 20°C gemessen.

12. Verfahren gemäß einem der Ansprüche 1 - 10 oder Kit nach Anspruch 11, **dadurch gekennzeichnet, dass** das mindestens eine Polymer, enthaltend (Meth)Acrylsäure-, (Meth)Acrylsäureester-, (Meth)Acrylamid- und/oder 2-Acrylamido-2-methylpropansulfonsäure-Monomere, ausgewählt ist aus vernetzten Homopolymeren der Acrylsäure, vernetzten Homopolymeren der 2-Acrylamido-2-methylpropansulfonsäure, vernetzten Copolymeren bestehend aus Acrylsäure- und 2-Acrylamido-2-methylpropansulfonsäure-Einheiten, vernetzten Copolymeren bestehend aus 2-Methyl-2[(1-oxo-2-propenyl)amino]-1-propansulfonsäure-Einheiten und Hydroxyethylacrylat-Einheiten, vernetzten Copolymeren bestehend aus Acrylamid- und 2-Acrylamido-2-methylpropansulfonsäure-Einheiten, vernetzten Copolymeren bestehend aus Methacrylsäure-Einheiten und C1-C4-Alkylacrylat-Einheiten, vernetzten Copolymeren bestehend aus Acrylsäure-Einheiten und C1-C4-Alkylmethacrylat-Einheiten, sowie Mischungen hiervon.

## Claims

1. A method for lightening and/or bleaching keratinous fibers, **characterized by** the following process steps:
a) providing a lightening or bleaching composition (A), containing:
a)i. at least one alkalizing agent;
a)ii. optionally at least one consistency enhancer; and
a)iii. optionally at least one solvent, selected from water and at least one organic solvent, preferably selected from one or more monovalent or polyvalent alcohols having from 2 to 9 carbon atoms, polyethylene glycols having from 2 to 20 ethylene glycol units, as well as mixtures of said solvents; and
a)iv. optionally at least one direct hair dye;
wherein, when the lightening composition (A), relative to its weight, contains more than 7 wt. % water, the lightening composition (A) has a pH value of from 6.0 to 12.0, preferably from 7.5 to 11.5, more particularly from 8 to 11.0, measured at 20° C., and
wherein when the lightening composition (A), relative to its weight, contains from 0 to 7 wt. % water, a 30 wt. % dispersion of the lightening composition (A) in water has a pH value of from 6.0 to 12.0, in each case measured at 20° C;
b) providing an oxidant composition (B), containing:
b)i. from 1 to 18 wt. % hydrogen peroxide, relative to the weight of the composition (B); and
b)ii. water;
b)iii. wherein the oxidant composition (B) has a pH value of from 2.5 to 6.5, preferably in the range from 3 to 5.5, more particularly in the range from 3.5 to 5.0, in each case measured at 20° C;
c) providing a polysaccharide composition (D), containing:
c)i. at least one polysaccharide, which can form a gel with calcium ions in a hydrous medium and is selected from alginic acid; kappa carragheenan, iota carragheenan and pectin, and/or at least one salt of the aforementioned polysaccharides, selected from the alkali metal-, ammonium-, mono-, di- and trialkyl ammonium-, mono-, di- and trialkanol ammonium- and magnesium salts, preferably in a total quantity of from 0.1 to 5 wt. %, preferably from 0.5 to 2 wt. %, more preferably from 0.5 to 2.5 wt. %, in each case relative to the weight of the composition (D), wherein in the case of the salts of kappa carragheenan, the alkali metal salt is selected only from lithium- and sodium salts;
c)ii. from 50 to 99.9 wt. % water, preferably from 60 to 95, more preferably from 80 to 90 wt. % water, in each case relative to the weight of the composition (D);
c)iii. optionally at least one alkalizing agent, which is preferably selected from powdery sodium silicates, more particularly sodium meta silicates;
c)iv. optionally at least one polymer, containing (meth)acrylic acid monomers, (meth)acrylic acid ester monomers, (meth)acrylamide monomers and/or 2-acrylamido-2-methylpropane sulfonic acid monomers, preferably in a total quantity of from 0.05 to 3 wt. %, more preferably from 0.2 to 0.5 wt. %, relative to the weight of the composition (D);
c)v. optionally at least one oil in a total quantity of from 0.1 to 10 wt. %, preferably from 0.3 to 5 wt. %, more preferably from 1 to 3 wt. %, in each case relative to the weight of the polysaccharide composition (D);
wherein optionally the polysaccharide composition (D) is produced by mixing a solid, preferably powdery, polysaccharide composition (D'), comprising c)i and optionally c)iii and/or optionally c)iv, with the component c)ii and optionally with the component c)v in situ or from 0.01 to 24 hours before applying the color-changing method as contemplated herein, subsequently, preferably from 1 to 600 seconds thereafter,
d) producing a mixture (M) of the lightening composition (A) and the oxidant composition (B), wherein the mixture (M) has a pH value of from 6.0 to 11, measured at 20° C; subsequently, after from 1 to 600 seconds
e) applying at least one partial quantity of (M) on at least one keratin fiber section to be bleached and/or lightened, or applying the agent (M) to the entire keratin fiber ensemble to be treated;
from 1 to 600 second, preferably from 5 to 300 seconds, more preferably from 5 to 60 seconds thereafter;
f) applying, preferably spraying, the polysaccharide composition (D) to the keratin fiber section(s) treated with (M) or the keratin fiber ensemble treated with;
g) optionally repeating steps e. and f. as many times as desired;
h) leaving on the keratinous fibers for a time of from 0.1 to 60 minutes, preferably from 1 to 50 minutes, more preferably from 10 to 45 minutes, most preferably from 30 to 45 minutes, and then rinsing the keratinous fibers with water and, if required, with a cleaning agent, post-treating the fibers with a conditioning agent and then drying, if required,
wherein the method comprises the application of at least one salt of a polyvalent metal ion, selected from calcium-, strontium-, barium- and aluminum salts having a water solubility at 20° C of at least 500 mg/l, and also in case the polysaccharide which forms a gel with calcium ions comprises kappa carragheenan or one of the salts thereof, the method comprises the application of at least one salt, selected from potassium-, calcium-, strontium-, barium- and aluminum salts having a water solubility at 20° C of at least 500 mg/l,
wherein these salts are not included in the polysaccharide composition (D).

2. The method of claim 1, wherein the bleaching or lightening composition (A) contains, relative to the weight of the composition (A), from 0.1 to 3 wt. %, preferably from 0.2 to 2 wt.-%, particularly preferred from 0.4 to 1 wt.-% of at least one salt of a polyvalent metal ion, selected from calcium salt, strontium salt, barium salt and aluminum salt having a water solubility at 20° C of at least 500 mg/l, and, if the polysaccharide, which can form a gel with Calcium ions, comprises kappa carragheenan or one of the salts thereof, the bleaching or lightening composition (A) contains, relative to the weight of the composition (A), from 0.1 to 3 wt. %, preferably from 0.2 to 2 wt.-%, preferably from 0.4 to 1 wt.-% of at least one salt, selected from potassium salts, calcium salts, strontium salts, barium salts and aluminum salts having a water solubility at 20° C of at least 500 mg/l.

3. The method of claim 1 or 2, wherein the oxidant composition (B) contains, relative to the weight of the oxidant composition (B), from 0.1 to 3 wt. %, preferably from 0.2 to 2 wt.-%, particularly preferred from 0.4 to 1 wt.-%, of at least one salt of a polyvalent metal ion, selected from calcium salt, strontium salt, barium salt and aluminum salt having a water solubility at 20° C of at least 500 mg/l, and, if the polysaccharide, which can form a gel with calcium ions, comprises kappa carragheenan or one of the salts thereof, the oxidant composition (B) contains, relative to the weight of the oxidant composition (B), from 0.1 to 3 wt. %, preferably from 0.2 to 2 wt.-%, particularly preferred from 0.4 to 1 wt.-%, of at least one salt, selected from potassium salts, calcium salts, strontium salts, barium salts and aluminum salts having a water solubility at 20° C of at least 500 mg/l.

4. The method according to any of claims 1 to 3, **characterized in that** the at least one salt of a polyvalent metal ion, selected from calcium salts, strontium salts, barium salts and aluminum salts having a water solubility at 20° C of at least 500 mg/l, and, if the polysaccharide, which can form a gel with calcium ions, comprises kappa carragheenan or one of the salts thereof, the at least one salt, selected from potassium salts, calcium salts, strontium salts, barium salts and aluminum salts having a water solubility at 20° C of at least 500 mg/l is provided in the form of a gelling composition (C) containing:
d)i. from 0.1 to 3 wt. %, preferably from 0.2 to 2 wt.-%, particularly preferred from 0.4 to 1 wt.-%, relative to the weight of the gel composition (C), of at least one salt of a polyvalent metal ion, selected from calcium salts, strontium salts, barium salts and aluminum salts having a water solubility at 20° C of at least 500 mg/l, and, if the polysaccharide, which forms a gel with calcium salts, comprises kappa carragheenan or one of the salts thereof, the gel composition (C) contains, relative to the weight of the gel composition (C), from 0.1 to 3 wt. %, preferably from 0.2 to 2 wt.-%, particularly preferred from 0.4 to 1 wt.-%, of at least one salt, selected from potassium salts, calcium salts, strontium salts, barium salts and aluminum salts having a water solubility at 20° C of at least 500 mg/l;
d)ii. from 50 to 99 wt. %, relative to the weight of the gel composition (C), water;
d)iii. optionally at least one cationic polymer, preferably selected from cationized guar ethers;
d)iv. wherein the gelling composition (C) has a pH value in the range from 4 to 12, preferably in the range 4 to 9, particularly preferred from 6.5 to 8, measured at 20° C;
wherein the gelling composition (C) is optionally produced in-situ or from 0.01 to 24 hours before executing the inventive bleaching or lightening process by mixing a solid gelling composition (C'), which preferably is in the form of a powder, containing the at least one salt cited under d)i. in powder form and optionally at least one cationic polymer, which is optionally selected from cationized guar ethers, in powder form, with water d)iii.;
**characterized in that**
- the gelling composition (C) is applied, preferably sprayed, onto the keratinous fiber(s), in a process step after the application step e and before applying, preferably spraying, the polysaccharide composition (D) onto the keratinous fiber(s), which carry the mixture (M) from (A) and (B); or
- the gelling composition (C) is added to one of the compositions (A) or (B), or to the mixture (M) from (A) and (B) before e) applying the mixture (M) on the selected keratinous fibers; or
- - the gelling composition (C) is applied, preferably sprayed, after applying, preferably spraying, the polysaccharide composition (D) onto the keratinous fiber(s), which carry the mixture (M) from (A) and (B).

5. The method according to any of claims 1 to 4, **characterized in that** the weight ratio of bleaching or lightening composition (A) to oxidant composition (B) is in the range from 1:10 to 4:1, preferably from 1:5 to 3:1, particularly preferred from 1:4 to 2:1 and extraordinarily preferred from 1:2 to 1:1.

6. The method according to claim 4 or 5, **characterized in that** the weight ratio of gelling composition (C) to mixture (M) from bleaching or lightening composition (A) and oxidant composition (B) is in the range from 1:20 to 1:2, preferably in the range from 1:10 to 1:5.

7. The method according to any of claims 1 to 6, **characterized in that** the weight ratio of polysaccharide composition (D) to mixture (M) from bleaching or lightening composition (A) and oxidant composition (B) is in the range from 1:20 to 1:2, preferably in the range from 1:10 to 1:5.

8. The method according to any of claims 4 to 7, **characterized in that** the weight ratio of polysaccharide composition (D) to gelling composition (C) is in the range from 0.2:1 to 2:1, preferably in the range from 0.5:1 to 1.5:1, and particularly preferred is 1:1.

9. The method according to any of claims 1 to 8, **characterized in that** said method is carried out with at least a second bleaching or lightening agent (A'), (A"), (A'") etc. instead of (A), wherein compositions (A) and (A'), (A"), (A'") etc. differ from one another in terms of quality and/or quantity, at least with respect to the direct hair dyes included therein.

10. The method according to any of claims 1 to 9, **characterized in that** the keratin fiber strands are not provided with solid, flat separating aids, more particularly not with foils from solid materials such as aluminum, paper or Styrofoam.

11. A kit for lightening and/or bleaching keratinous fibers comprising the following components (D) and (C) and optionally (A) and optionally (B), which are physically separated from one another:
I. a polysaccharide composition (D), comprising:
d)i. at least one polysaccharide, which is able to form a gel with calcium ions in a hydrous medium and which is selected from alginic acid; kappa-carragheenan, iota carragheenan; and pectin; and/or at least one salt of said polysaccharides, selected from alkali metal salts, ammonium salts, mono-, di- and trialkyl ammonium salts, mono-, di- and trialkanol ammonium salts, and magnesium salts; wherein for kappa-carragheenan its alkali metal salts are only selected from lithium salt and sodium salt; wherein mixtures of alginic acid and sodium alginate are particularly preferred;
d)ii. at least one alkalizing agent, which is preferably selected from powdery sodium silicates, more particularly powdery sodium meta silicates;
d)iii. optionally at least one polymer containing (meth)acrylic acid monomers, (meth)acrylic acid ester monomers, (meth)acrylamide- and/or 2-acrylamido-2-methylpropane sulfonic acid monomers, wherein the at least one polymer preferably does not comprise monomers which are substituted with, optionally ethoxylated, C6-C24-alkyl- or C6-C24-alkenyl groups;
II. a gelling composition (C'), comprising:
c)i. at least one salt of a polyvalent metal ion, selected from calcium salt, strontium salt, barium salt and aluminum salts having a water solubility at 20° C of at least 500 mg/l, and, if the polysaccharide capable of forming a gel with calcium ions comprises kappa carragheenan or one of the salts thereof, at least one salt, selected from potassium salts, calcium salts, strontium salts, barium salts and aluminum salts having a water solubility at 20° C of at least 500 mg/l; and
c)ii. optionally at least one cationic polymer, preferably selected from cationized guar ethers;
III. optionally a bleaching or lightening composition (A), comprising:
a)i. at least one alkalizing agent;
a)ii. optionally at least one consistency enhancer;
a)iii. optionally at least one solvent, selected from water, one or more mono- or polyvalent alcohols having from 2 to 9 carbon atoms, and mixtures thereof; and
a)iv. optionally at least one direct hair dye;
wherein when the lightening composition (A), relative to its weight, contains more than 7 wt. % water, said composition (A) has a pH value of from 6.0 to 11.5, measured at 20° C, and wherein when the lightening composition (A), relative to its weight, contains from 0 to 7 wt. % water, a 30 wt. % dispersion of said composition (A) in water has a pH value of from 6.0 to 12.0, preferably from 7.5 to 11.5, more preferably from 8 to 11.0, in each case measured at 20° C; and
IV. optionally an oxidant composition (B), comprising:
b)i. from 1 to 18 wt. % hydrogen peroxide, relative to the weight of the composition (B); and
b)ii. water;
b)iii. wherein the oxidant composition (B) has a pH value of from 2.5 to 6.5, preferably from 3 to 5.5, more preferably from 3.5 to 5.0, in each case measured at 20° C.

12. The method of any of claims 1 to 10 or the kit according to claim 11, **characterized in that** the at least one polymer containing (meth)acrylic acid monomers, (meth)acrylic acid ester monomers, (meth)acrylamide- and/or 2-acrylamido-2-methylpropane sulfonic acid monomers, is selected from cross-linked homopolymers of acrylic acid, cross-linked homopolymers of 2-acrylamido-2-methylpropane sulfonic acid, cross-linked copolymers consisting of acrylic acid- and 2-acrylamido-2-methylpropane sulfonic acid units, cross-linked copolymers consisting of 2-methyl-2[(1-oxo-2-propenyl)amino]-1-propane sulfonic acid units and hydroxyethylacrylate units, cross-linked copolymers consisting of acrylamide- and 2-acrylamido-2-methylpropane sulfonic acid units, cross-linked copolymers consisting of methacrylic acid units and C1-C4-alkylacrylate units, cross-linked copolymers consisting of acrylic acid units and C1-C4-alkylmethacrylate units, as well as mixtures thereof.

## Revendications

1. Procédé pour éclaircir et/ou blondir les fibres kératiniques, **caractérisé par** les étapes de procédé suivantes :
a. fourniture d'une composition de blondissement ou d'éclaircissement (A) contenant
a)i au moins un agent alcalinisant,
a)ii éventuellement au moins un agent de consistance, et
a)iii éventuellement au moins un solvant choisi parmi l'eau et au moins un solvant organique, de préférence choisi parmi un ou plusieurs alcools monovalents ou polyvalents comportant 2 à 9 atomes de carbone, des polyéthylèneglycols comportant 2 à 20 unités d'éthylèneglycol ainsi que des mélanges de ces solvants, et
a)iv éventuellement au moins une teinture capillaire directe,
a)v la composition de blondissement ou d'éclaircissement (A), dans la mesure où elle contient plus de 7 % en poids d'eau par rapport à son poids, présente un pH dans la plage allant de 6,0 à 11,5, mesuré à 20 °C, et dans la mesure où la composition (A) contient de 0 à 7 % en poids d'eau par rapport à son poids, sa dispersion de 30 % en poids dans l'eau présente un pH dans la plage allant de 6,0 à 12,0, de préférence de 7,5 à 11,5, de manière particulièrement préférée de 8 à 11,0, mesuré dans chaque cas à 20 °C ;
b. fourniture d'une composition d'oxydation (B) contenant
b)i de 1 à 18 % en poids de peroxyde d'hydrogène, par rapport au poids de la composition (B), et
b)ii de l'eau,
b)iii la composition d'oxydation (B) présentant un pH dans la plage allant de 2,5 à 6,5, de préférence dans la plage allant de 3 à 5,5, de manière particulièrement préférée dans la plage allant de 3,5 à 5,0, mesuré dans chaque cas à 20 °C ;
c. fourniture d'une composition de polysaccharide (D) contenant
c)i au moins un polysaccharide pouvant former un gel avec des ions calcium en milieu aqueux et choisi parmi l'acide alginique, le kappa-carragheenan, l'iota-carragheenan et la pectine, et/ou au moins un sel des polysaccharides précités, choisi parmi les sels de métal alcalin, les sels d'ammonium, les sels de mono-, di- et trialkylammonium, les sels de mono-, di- et trialkanolammonium et les sels de magnésium, de préférence en une quantité totale de 0,1 à 5 % en poids, de préférence de 0,5 à 2 % en poids, de manière particulièrement préférée de 0,5 à 2,5 % en poids, dans chaque cas par rapport au poids de la composition (D), le sel de métal alcalin étant choisi uniquement parmi les sels de lithium et de sodium dans le cas des sels de kappa-carragheenan,
c)ii de 50 à 99,9, de préférence de 60 à 95, de manière particulièrement préférée de 80 à 90 % en poids d'eau, dans chaque cas par rapport au poids de la composition (D),
c)iii éventuellement au moins un agent alcalinisant, de préférence choisi parmi des silicates de sodium en poudre, en particulier des métasilicates de sodium en poudre,
c)iv éventuellement au moins un polymère contenant des monomères d'acide (méth)acrylique, des monomères d'ester (méth)acrylique, des monomères de (méth)acrylamide et/ou des monomères d'acide 2-acrylamido-2-méthylpropanesulfonique, de préférence en une quantité totale de 0,05 à 3 % en poids, de préférence 0,2 à 0,5 % en poids, par rapport au poids de la composition (D),
c)v éventuellement au moins une huile en une quantité totale de 0,1 à 10 % en poids, de préférence de 0,3 à 5 % en poids, de manière particulièrement préférée de 1 à 3 % en poids, dans chaque cas par rapport au poids de la composition de polysaccharide (D),
dans lequel, éventuellement, la composition de polysaccharide (D) est préparée *in situ* ou 0,01 à 24 heures avant la mise en œuvre du procédé de changement de couleur selon l'invention, en mélangeant une composition de polysaccharide (D') solide, de préférence pulvérulente contenant c)i et éventuellement c)iii et/ou éventuellement c)iv avec le composant c)ii et éventuellement avec le composant c)v, puis, de préférence 1 à 600 secondes après,
d. préparation d'un mélange (M) de (A) et (B) qui présente un pH dans la plage allant de 6,0 à 11, mesuré dans chaque cas à 20 °C ; après 1 à 600 secondes, puis
e. application d'au moins une quantité partielle de (M) à au moins une partie des fibres kératiniques à éclaircir et/ou à blondir ou application de l'agent (M) à l'ensemble des fibres kératiniques à traiter ;
1 à 600 secondes, de préférence de 5 à 300 secondes, de manière particulièrement préférée de 5 à 60 secondes après,
f. application, de préférence par pulvérisation, de la composition de polysaccharide (D) sur la/les partie(s) des fibres kératiniques sur lesquelles (M) est appliqué ou sur l'ensemble des fibres kératiniques sur lesquelles (M) est appliqué ;
g. éventuellement, répétition des étapes e. et f. dans la mesure souhaitée,
h. pendant une durée de 0,1 à 60 minutes, de préférence de 1 à 50 minutes, de manière particulièrement préférée de 10 à 45 minutes, de manière exceptionnellement préférée de 30 à 45 minutes, maintien de l'agent sur les fibres kératiniques, puis rinçage des fibres kératiniques avec de l'eau et éventuellement un agent nettoyant, éventuellement, post-traitement des fibres avec un agent de conditionnement et le séchage ultérieur,
**caractérisé en ce que** le procédé comprend l'application d'au moins un sel d'un ion métallique polyvalent choisi parmi les sels de calcium, de strontium, de baryum et d'aluminium avec une solubilité dans l'eau à 20 °C d'au moins 500 mg/l, et dans le cas où le polysaccharide formant un gel avec des ions calcium comprend du kappa-carragheenan ou l'un de ses sels, le procédé comprend l'application d'au moins un sel choisi parmi les sels de potassium, de calcium, de strontium, de baryum et d'aluminium avec une solubilité dans l'eau à 20 °C d'au moins 500 mg/l,
ces sels n'étant pas contenus dans la composition de polysaccharide (D).

2. Procédé selon la revendication 1, **caractérisé en ce que** la composition de blondissement ou d'éclaircissement (A) contient de 0,1 à 3 % en poids, de préférence de 0,2 à 2 % en poids, de manière particulièrement préférée de 0,4 à 1 % en poids, par rapport au poids de la composition (A), d'au moins un sel d'un ion métallique polyvalent choisi parmi les sels de calcium, de strontium, de baryum et d'aluminium avec une solubilité dans l'eau à 20 °C d'au moins 500 mg/l, et dans le cas où le polysaccharide formant un gel avec des ions calcium comprend du kappa-carragheenan ou l'un de ses sels, la composition de blondissement ou d'éclaircissement (A) contient de 0,1 à 3 % en poids, de préférence de 0,2 à 2 % en poids, de manière particulièrement préférée de 0,4 à 1 % en poids, par rapport au poids de la composition (A), d'au moins un sel choisi parmi les sels de potassium, de calcium, de strontium, de baryum et d'aluminium avec une solubilité dans l'eau à 20 °C d'au moins 500 mg/l.

3. Procédé selon la revendication 1 ou 2, **caractérisé en ce que** la composition d'oxydation (B) contient de 0,1 à 3 % en poids, de préférence de 0,2 à 2 % en poids, de manière particulièrement préférée de 0,4 à 1 % en poids, par rapport au poids de la composition (B), d'au moins un sel d'un ion métallique polyvalent choisi parmi les sels de calcium, de strontium, de baryum et d'aluminium avec une solubilité dans l'eau à 20 °C d'au moins 500 mg/l, et dans le cas où le polysaccharide formant un gel avec des ions calcium comprend du kappa-carragheenan ou l'un de ses sels, la composition d'oxydation (B) contient de 0,1 à 3 % en poids, de préférence de 0,2 à 2 % en poids, de manière particulièrement préférée de 0,4 à 1 % en poids, par rapport au poids de la composition (B), d'au moins un sel choisi parmi les sels de potassium, de calcium, de strontium, de baryum et d'aluminium avec une solubilité dans l'eau à 20 °C d'au moins 500 mg/l.

4. Procédé selon l'une des revendications 1 à 3, **caractérisé en ce que** l'au moins un sel d'un ion métallique polyvalent choisi parmi les sels de calcium, de strontium, de baryum et d'aluminium avec une solubilité dans l'eau à 20 °C d'au moins 500 mg/l, et dans le cas où le polysaccharide formant un gel avec des ions calcium comprend du kappa-carragheenan ou l'un de ses sels, l'au moins un sel d'un ion métallique polyvalent choisi parmi les sels de potassium, de calcium, de strontium, de baryum et d'aluminium avec une solubilité dans l'eau à 20 °C d'au moins 500 mg/l est fourni sous la forme d'une composition gélifiante (C), contenant
d)i de 0,1 à 3 % en poids, de préférence de 0,2 à 2 % en poids, de manière particulièrement préférée de 0,4 à 1 % en poids, par rapport au poids de la composition (C), d'au moins un sel d'un ion métallique polyvalent choisi parmi les sels de calcium, de strontium, de baryum et d'aluminium avec une solubilité dans l'eau à 20 °C d'au moins 500 mg/l, et dans le cas où le polysaccharide formant un gel avec des ions calcium comprend du kappa-carragheenan ou l'un de ses sels, la composition gélifiante (C) contient de 0,1 à 3 % en poids, de préférence de 0,2 à 2 % en poids, de manière particulièrement préférée de 0,4 à 1 % en poids, par rapport au poids de la composition (C), d'au moins un sel choisi parmi les sels de potassium, de calcium, de strontium, de baryum et d'aluminium avec une solubilité dans l'eau à 20 °C d'au moins 500 mg/l,
d)ii de 50 à 99 % en poids d'eau, par rapport au poids de la composition (C),
d)iii éventuellement au moins un polymère cationique, de préférence choisi parmi les éthers de guar cationisés ;
d)iv la composition gélifiante (C) présentant une valeur de pH dans la plage allant de 4 à 12, de préférence dans la plage allant de 4 à 9, de manière particulièrement préférée de 6,5 à 8, mesuré à 20 °C ; la composition gélifiante (C) étant préparée *in situ* ou 0,01 à 24 heures avant la mise en œuvre du procédé de blondissement ou d'éclaircissement selon l'invention, en mélangeant une composition gélifiante (C') solide, de préférence pulvérulente, contenant l'au moins un sel mentionné sous d)i sous forme de poudre et éventuellement au moins un polymère cationique, de préférence choisi parmi les éthers de guar cationisés, sous forme de poudre, avec de l'eau d)iii,
**caractérisé en ce que** la composition gélifiante (C), dans une étape du procédé,
- est appliquée sur la/les fibre(s) kératinique(s) sur laquelle/lesquelles le mélange (M) de (A) et (B) a été appliqué, de préférence vaporisé, après l'étape d'application e et avant l'application, de préférence par pulvérisation, de la composition de polysaccharide (D), ou
- est ajoutée à l'une des compositions (A) ou (B) ou au mélange (M) de (A) et (B) avant l'étape d'application e, ou
- est appliquée, de préférence pulvérisée sur la/les fibre(s) kératinique(s) sur laquelle/lesquelles le mélange (M) de (A) et (B) a été appliqué, après l'application, de préférence par pulvérisation, de la composition de polysaccharide (D).

5. Procédé selon l'une des revendications 1 à 4, **caractérisé en ce que** le rapport pondéral entre la composition de blondissement ou d'éclaircissement (A) et la composition d'oxydation (B) se situe dans la plage allant de 1:10 à 4:1, de préférence dans la plage allant de 1:5 à 3:1, de manière particulièrement préférée dans la plage allant de 1:4 à 2:1 et de manière exceptionnellement préférée dans la plage allant de 1:2 à 1:1.

6. Procédé selon la revendication 4 ou 5, **caractérisé en ce que** le rapport pondéral entre la composition gélifiante (C) et le mélange (M) de la composition de blondissement ou d'éclaircissement (A) et de la composition d'oxydation (B) se situe dans la plage allant de 1:20 à 1:2, de préférence dans la plage allant de 1:10 à 1:5.

7. Procédé selon l'une des revendications 1 à 6, **caractérisé en ce que** le rapport pondéral entre la composition de polysaccharide (D) et le mélange (M) de la composition de blondissement ou d'éclaircissement (A) et de la composition d'oxydation (B) se situe dans la plage allant de 1:20 à 1:2, de préférence dans la plage allant de 1:10 à 1:5.

8. Procédé selon l'une des revendications 4 à 7, **caractérisé en ce que** le rapport pondéral entre la composition de polysaccharide (D) et la composition gélifiante (C) se situe dans la plage allant de 0,2:1 à 2:1, de préférence dans la plage allant de 0,5:1 à 1,5:1, et est de manière particulièrement préférée de 1:1.

9. Procédé selon l'une des revendications 1 à 8, **caractérisé en ce que** l'on procède avec au moins une seconde composition de blondissement ou d'éclaircissement (A'), (A"), (A"'), etc. au lieu de (A), les compositions (A) et (A'), (A"), (A"'), etc. différant qualitativement et/ou quantitativement les unes des autres au moins en ce qui concerne les teintures capillaires directes présentes.

10. Procédé selon l'une des revendications 1 à 9, **caractérisé en ce que** les brins de fibres kératiniques ne sont pas pourvus d'auxiliaires de séparation plats solides, ne sont notamment pas pourvus de feuilles en matériaux solides tels que l'aluminium, le papier ou le polystyrène.

11. Kit pour éclaircir et/ou blondir les fibres kératiniques, comprenant les composants (D) et (C) ci-après, physiquement séparés l'un des l'autre, ainsi qu'éventuellement (A) et éventuellement (B) :
I. une composition de polysaccharide (D) contenant
d)i au moins un polysaccharide pouvant former un gel avec des ions calcium en milieu aqueux et choisi parmi l'acide alginique, le kappa-carragheenan, l'iota-carragheenan et la pectine, et/ou au moins un sel des polysaccharides précités, choisi parmi les sels de métal alcalin, d'ammonium, de mono-, di- et trialkylammonium, de mono-, di- et trialkanolammonium et de magnésium, le sel de métal alcalin étant choisi uniquement parmi les sels de lithium et de sodium, dans le cas des sels de kappa-carragheenan, son sel de métal alcalin étant choisi uniquement parmi les sels de lithium et de sodium, les mélanges de sels alginiques et d'alginate de sodium étant particulièrement préférés,
d)ii au moins un agent alcalinisant, de préférence choisi parmi les silicates de sodium en poudre, en particulier les métasilicates de sodium en poudre,
d)iii éventuellement au moins un polymère contenant des monomères d'acide (méth)acrylique, d'ester d'acide (méth)acrylique, de (méth)acrylamide et/ou d'acide 2-acrylamido-2-méthylpropanesulfonique, lequel polymère ne présente de préférence pas de monomères, lesquels monomères sont éventuellement substitués par des groupes alkyle en C₆-C₂₄ ou alcényle en C₆-C₂₄ éthoxylés ;
II. une composition gélifiante (C') contenant
c)i au moins un sel d'un ion métallique polyvalent choisi parmi les sels de calcium, de strontium, de baryum et d'aluminium avec une solubilité dans l'eau à 20 °C d'au moins 500 mg/l, et dans le cas où le polysaccharide formant un gel avec des ions calcium comprend un kappa-carragheenan ou l'un de ses sels, au moins un sel choisi parmi les sels de potassium, de calcium, de strontium, de baryum et d'aluminium avec une solubilité dans l'eau à 20 °C d'au moins 500 mg/l ;
c)ii éventuellement au moins un polymère cationique, de préférence choisi parmi les éthers de guar cationisés,
III. éventuellement une composition de blondissement ou d'éclaircissement (A) contenant
a)i au moins un agent alcalinisant,
a)ii éventuellement au moins un agent de consistance, et
a)iii éventuellement au moins un solvant choisi parmi l'eau, un ou plusieurs alcools monovalents ou polyvalents comportant 2 à 9 atomes de carbone ainsi que des mélanges de ces solvants, et
a)iv éventuellement au moins une teinture capillaire directe,
la composition de blondissement ou d'éclaircissement (A), dans la mesure où elle contient plus de 7 % en poids d'eau par rapport à son poids, présente un pH dans la plage allant de 6,0 à 11,5, mesuré à 20 °C, et dans la mesure où la composition (A) contient de 0 à 7 % en poids d'eau par rapport à son poids, sa dispersion de 30 % en poids dans l'eau présente un pH dans la plage allant de 6,0 à 12,0, de préférence de 7,5 à 11,5, de manière particulièrement préférée de 8 à 11,0, mesuré dans chaque cas à 20 °C ;
IV. éventuellement une composition d'oxydation (B) contenant
b)i de 1 à 18 % en poids de peroxyde d'hydrogène, par rapport au poids de la composition (B), et
b)ii de l'eau,
b)iii la composition d'oxydation (B) présentant un pH dans la plage allant de 2,5 à 6,5, de préférence dans la plage allant de 3 à 5,5, de manière particulièrement préférée dans la plage allant de 3,5 à 5,0, mesuré dans chaque cas à 20 °C.

12. Procédé selon l'une des revendications 1 à 10 ou kit selon la revendication 11, **caractérisé en ce que** l'au moins un polymère contenant des monomères d'acide (méth)acrylique, d'ester d'acide (méth)acrylique, de (méth)acrylamide et/ou d'acide 2-acrylamido-2-méthylpropanesulfonique est choisi parmi les homopolymères réticulés d'acide acrylique, les homopolymères réticulés d'acide 2-acrylamido-2-méthylpropanesulfonique, les copolymères réticulés constitués de motifs acide acrylique et de motifs acide 2-acrylamido-2-méthylpropanesulfonique, les copolymères réticulés constitués de motifs acide 2-méthyl-2[(1-oxo-2-propenyl)amino]-1-propanesulfonique et de motifs hydroxyéthylacrylate, les copolymères réticulés constitués de motifs acrylamide et de motifs acide 2-acrylamido-2-méthylpropanesulfonique, les copolymères réticulés constitués de motifs acide méthacrylique et de motifs acrylate d'alkyle en C₁-C₄, les copolymères réticulés constitués de motifs acide acrylique et de motifs méthacrylate d'alkyle en C₁-C₄, ainsi que des mélanges de ceux-ci.
